(19) Europäisches Patentamt
European Patent Office
Office européen des brevets

(11) Veröffentlichungsnummer: **0 433 648 A1**

(12) **EUROPÄISCHE PATENTANMELDUNG**

(21) Anmeldenummer: **90121665.5**

(22) Anmeldetag: **13.11.90**

(51) Int. Cl.[5]: **C07D 487/04**, C07C 275/40, C07D 235/16, //(C07D487/04, 239:00,235:00)

(30) Priorität: **21.11.89 CH 4165/89**
**17.08.90 CH 2688/90**
**30.08.90 CH 2817/90**

(43) Veröffentlichungstag der Anmeldung:
**26.06.91 Patentblatt 91/26**

(84) Benannte Vertragsstaaten:
**AT BE CH DE DK FR GB IT LI LU NL SE**

(71) Anmelder: **F. HOFFMANN-LA ROCHE AG**
**Postfach 3255**
**CH-4002 Basel(CH)**

(72) Erfinder: **Hubschwerlen, Christian, Dr.**
**Rue de la Gendarmerie**
**F-68200 Durmenach(FR)**
Erfinder: **Kompis, Ivan, Dr.**
**Goldentalweg 16**
**CH-4104 Oberwil(CH)**
Erfinder: **Specklin, Jean-Luc**
**Rue du 6ème Ric**
**F-68680 Kembs-Loechle(FR)**

(74) Vertreter: **Martin, Björn et al**
**Grenzacherstrasse 124 Postfach 3255**
**CH-4002 Basel(CH)**

(54) **Substituierte Pyrimidobenzimidazolderivate.**

(57) Die vorliegende Erfindung betrifft neue substituierte Pyrimidobenzimidazolderivate der allgemeinen Formel

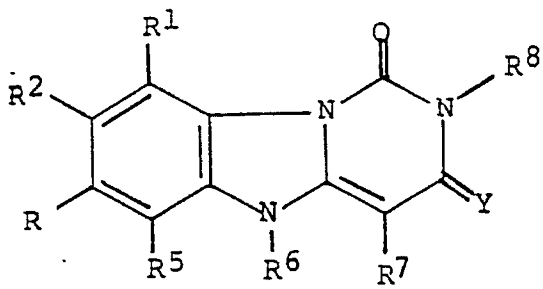

I

worin $R^1$ Wasserstoff, Halogen oder Amino, $R^2$ Halogen, R eine durch niederes Alkyl substituierte 4-Pyridylgruppe oder eine Gruppe $R^3R^4N$-, worin $R^3$ und $R^4$ je Wasserstoff oder niederes Alkyl oder zusammen eine unsubstituierte oder durch niederes Alkyl, Amino, niederes Aminoalkyl, mono- oder di(niederes Alkyl)amino-niederes Alkyl, Oxo oder die Gruppe -COOR$^a$ oder -CONR$'$R$''$ substituierte Gruppe der Formel $-(CH_2)_n-X-(CH_2)_m-$ oder $-(CH_2)_p-$, n und m je die Zahl 1, 2 oder 3, wobei n + m höchstens 5 beträgt, p die Zahl 4, 5 oder 6, X ein Sauerstoff- oder Schwefelatom oder die Gruppe -NR$'''$-, R$^a$ Wasserstoff, niederes Alkyl, niederes Alkenyl, Phenyl oder durch Halogen, niederes Alkyl oder Hydroxy mono-, di- oder trisubstituiertes Phenyl, R$'$ und R$''$ je Wasserstoff oder niederes Alkyl, R$'''$ Wasserstoff, Hydroxy, niederes Alkyl oder niederes Aminoalkanoyl, $R^5$ Wasserstoff, Halogen, niederes Alkoxy oder Amino, $R^6$ niederes Alkyl, niederes Cycloalkyl, niederes Halogenalkyl, Phenyl oder durch Halogen, niederes Alkyl, Hydroxy oder niederes Alkoxy mono-, di- oder trisubstituiertes Phenyl, $R^7$ Wasserstoff, niederes Alkyl oder Carboxy, $R^8$ Wasserstoff, Hydroxy, niederes Alkoxy, Amino, niederes Alkylamino oder di-niederes Alkylamino und Y ein Sauerstoff- oder Schwefelatom bedeuten,

EP 0 433 648 A1

und pharmazeutisch annehmbare Salze davon.

Die Produkte besitzen eine hemmende Wirkung auf die DNA-Gyrase-Aktivität in Bakterien. Sie können zur Verhütung oder Bekämpfung von bakteriellen Infektionen verwendet werden.

# SUBSTITUIERTE PYRIMIDOBENZIMIDAZOLDERIVATE

Die vorliegende Erfindung betrifft neue substituierte Pyrimidobenzimidazolderivate der allgemeinen Formel

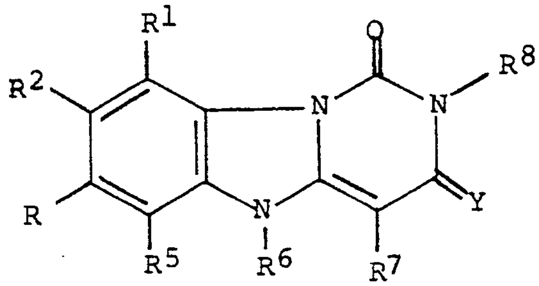

I

worin $R^1$ Wasserstoff, Halogen oder Amino, $R^2$ Halogen, R eine durch niederes Alkyl substituierte 4-Pyridylgruppe oder eine Gruppe $R^3R^4N$-, worin $R^3$ und $R^4$ je Wasserstoff oder niederes Alkyl oder zusammen eine unsubstituierte oder durch niederes Alkyl, Amino, niederes Aminoalkyl, mono- oder di-(niederes Alkyl)amino-niederes Alkyl, Oxo oder die Gruppe $-COOR^a$ oder $-CONR'R''$ substituierte Gruppe der Formel $-(CH_2)_n-X(CH_2)_m-$ oder $-(CH_2)_p-$, n und m je die Zahl 1, 2 oder 3, wobei n + m höchstens 5 beträgt, p die Zahl 4, 5 oder 6, X ein Sauerstoff- oder Schwefelatom oder die Gruppe $-NR'''-$, $R_a$ Wasserstoff, niederes Alkyl, niederes Alkenyl, Phenyl oder durch Halogen, niederes Alkyl oder Hydroxy mono-, di- oder trisubstituiertes Phenyl, $R'$ und $R''$ je Wasserstoff oder niederes Alkyl, $R'''$ Wasserstoff, Hydroxy, niederes Alkyl oder niederes Aminoalkanoyl, $R^5$ Wasserstoff, Halogen, niederes Alkoxy oder Amino, $R^6$ niederes Alkyl, niederes Cycloalkyl, niederes Halogenalkyl, Phenyl oder durch Halogen, niederes Alkyl, Hydroxy oder niederes Alkoxy mono-, di- oder trisubstituiertes Phenyl, $R^7$ Wasserstoff, niederes Alkyl oder Carboxy, $R^8$ Wasserstoff, Hydroxy, niederes Alkoxy, Amino, niederes Alkylamino oder di-niederes Alkylamino und Y ein Sauerstoff- oder Schwefelatom bedeuten,
und pharmazeutisch annehmbare Salze davon.

Die neuen Verbindungen der obigen Formel I besitzen wertvolle pharmakologische Eigenschaften. Sie entfalten eine hemmende Wirkung auf die DNA-Gyrase-Aktivität in Bakterien und können daher zur Bekämpfung oder Verhütung von bakteriellen Infektionen verwendet werden.

Gegenstand der vorliegenden Erfindung sind: Die obigen Verbindungen der Formel I als solche und zur Anwendung als therapeutische Wirkstoffe; ein Verfahren und Zwischenprodukte zu deren Herstellung, Arzneimittel auf der Basis dieser neuen Stoffe; sowie die Verwendung der neuen Verbindungen der Formel I zur Bekämpfung oder Verhütung von bakteriellen Infektionen und zur Herstellung von antibakteriell wirksamen Mitteln.

Der im Rahmen der vorliegenden Beschreibung verwendete Ausdruck "nieder" bezeichnet Reste und Verbindungen mit höchstens 7, vorzugzsweise höchstens 4 Kohlenstoffatomen. Der Ausdruck "Alkyl" für sich allein genommen oder in Zusammensetzungen, wie "Alkylgruppe" und "Alkoxy", bezeichnet geradkettige oder verzweigte, gesättigte Kohlenwasserstoffreste, wie Methyl, Aethyl, n-Propyl, i-Propyl, n-Butyl, s-Butyl, i-Butyl und t-Butyl. Der Ausdruck "Alkenyl" bezeichnet geradkettige oder verzeigte Kohlenwasserstoffreste, welche mindestens eine olefinische Doppelbindung enthalten, wie Allyl und 2-Butenyl. Der Ausdruck "Cycloalkyl" bezeichnet cyclische gesättigte Kohlenwasserstoffreste, wie Cyclopropyl. Der Ausdruck "Alkanoyl" bezeichnet Reste von geradkettigen oder verzweigten, gesättigten Fettsäuren, wie Acetyl. Der Ausdruck "Halogen" bezeichnet die vier Formen Fluor, Chlor, Brom und Jod.

$R^1$ und $R^5$ bedeuten vorzugsweise je Wasserstoff. $R^2$ bedeutet vorzugsweise Fluor. $R^3$ und $R^4$ bedeuten zusammen vorzugsweise eine Gruppe der Formel $(CH_2)_n-X-(CH_2)_m$-oder eine durch die Gruppe $-COOR^a$ substituierte Gruppe der Formel $-(CH_2)_p-$, wobei n und m je die Zahl 2, p die Zahl 4, X die Gruppe $-NR'''-$, $R^a$ niederes Alkyl und $R'''$ Wasserstoff, niederes Alkyl oder niederes Aminoalkanoyl bedeuten. $R^3R^4N$- ist vorzugsweise 1-Piperazinyl oder 4-Methyl-1-piperazinyl. $R^6$ bedeutet vorzugsweise niederes Alkyl, insbesondere Aethyl, oder niederes Cycloalkyl, insbesondere Cyclopropyl. $R^7$ bedeutet vorzugsweise Wasserstoff oder Carboxy. $R^8$ bedeutet vorzugsweise Wasserstoff, Hydroxy, Amino, Methylamino oder Dimethylamino. Y bedeutet vorzugsweise ein Sauerstoffatom. R in der Bedeutung als nieder-alkylsubstituiertes 4-Pyridyl stellt vorzugsweise die 3,5-Dimethyl-4-pyridylgruppe dar.

Die nachfolgend aufgeführten Verbindungen sind repräsentative Vertreter der durch die allgemeinen Formel I definierten neuen Stoffklasse:

5-Aethyl-8-fluor-7-(4-methyl-1-piperazinyl)-pyrimido[1.6-a]benzimidazol-1,3(2H,5H)-dion,
5-Cyclopropyl-8-fluor-7-(1-piperazinyl)-pyrimido[1,6-a]benzimidazol-1,3(2H,5H)-dion,
5-Aethyl-9-fluor-3,5-dihydro-8-(4-methyl-1-piperazinyl)-3-thioxopyrimido[1,6-a]benzimidazol-1(2H)-on,
5-Cyclopropyl-8-fluor-2-hydroxy-7-(1-piperazinyl)pyrimido[1,6-a]benzimidazol-1,3(2H,5H)-dion,
tert-Butyl rac-1-(5-cyclopropyl-8-fluor-2,3-dihydro-1,3-dioxopyrimido[1,6-a]benzimidazol-5(1H)-yl]-2-pyrrolidincarboxylat,
7-(4-L-Alanyl-1-piperazinyl)-5-cyclopropyl-8-fluor-2-hydroxypyrimido[1,6-a]benzimidazol-1,3(2H,5H)-dion,
1-[5-Cyclopropyl-8-fluor-1,2,3,5-tetrahydro-2-hydroxy-1,3-dioxopyrimido[1,6-a]benzimidazol-7-yl]-L-prolin-tert-butylester,
5-Aethyl-8-fluor-7-(1-piperazinyl)pyrimido[1,6-a]benzimidazol-1,3(2H,5H)-dion,
5-Aethyl-8-fluor-2-hydroxy-7-(4-methyl-1-piperazinyl)pyrimido[1,6-a]benzimidazol-1,3(2H,5H)-dion,
5-Cyclopropyl-8-fluor-1,2,3,4-tetrahydro-1,3-dioxo-7-(1-piperazinyl)pyrimido[1,6-a]benzimidazol-4-carbonsäure,
5-Aethyl-8-fluor-2-hydroxy-7-(1-piperazinyl)pyrimido[1,6-a]benzimidazol-1,3(2H,5H)-dion,
2-Amino-5-cyclopropyl-8-fluor-7-(1-piperazinyl)-pyrimido[1,6-a]benzimidazol-1,3(2H,5H)-dion,
5-Cyclopropyl-8-fluor-2-(methylamino)-7-(1-piperazinyl)pyrimido[1,6-a]benzimidazol-1,3(2H,5H)-dion und
5-Cyclopropyl-2-(dimethylamino)-8-fluor-7-(1-piperazinyl)pyrimido[1,6-a]benzimidazol-1,3(2H,5H)-dion.

Die neuen Verbindungen der Formel I und ihre pharmazeutisch annehmbaren Salze können erfindungsgemäss dadurch hergestellt werden, dass man

a) eine Verbindung der allgemeinen Formel

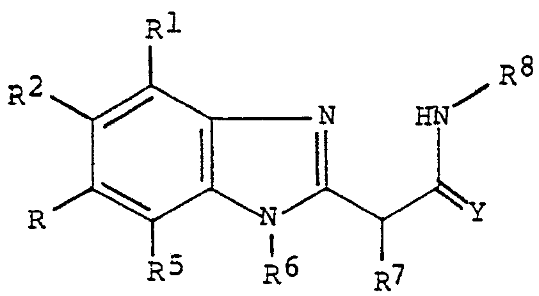

II

worin R, Y, $R^1$, $R^2$, $R^5$, $R^6$, $R^7$ und $R^8$ obige Bedeutung besitzen, wobei allfällig vorhandene freie Hydroxy-, Amino- und Carboxygruppen in geschützter Form vorliegen,
gegebenenfalls in Gegenwart einer Base mit einer Verbindung der allgemeinen Formel
X-CO-X III
worin X eine Abgangsgruppe bedeutet,
umsetzt, oder

b) eine Verbindung der allgemeinen Formel

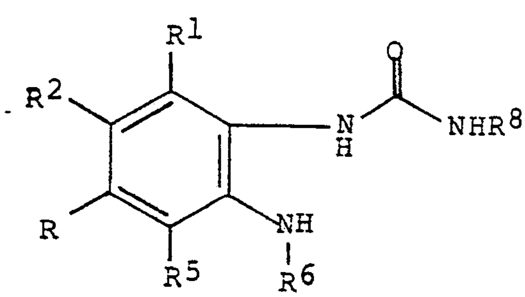

IV

worin R, $R^1$, $R^2$, $R^5$, $R^6$ und $R^8$ obige Bedeutung besitzen, wobei allfällig vorhandene freie Hydroxy-, Amino- und Carboxygruppen in geschützter Form vorliegen,

in Gegenwart einer Base mit einer Verbindung der allgemeinen Formel

RaOOC-CHR$^7$-COORa V

worin Ra niederes Alkyl bedeutet, und R$^7$ obige Bedeutung besitzt, wobei eine allfällig vorhandene freie Carboxygruppe in geschützter Form vorliegt,

umsetzt, oder

c) eine Verbindung der allgemeinen Formel

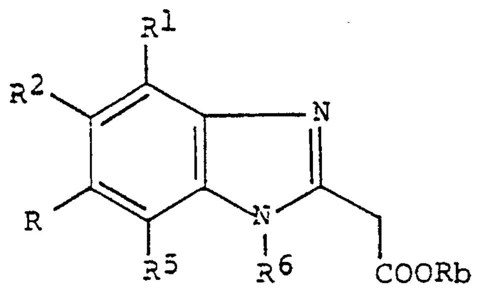

VI

worin Rb eine Carboxy-Schutzgruppe bedeutet, und R, R$^1$, R$^2$, R$^5$ und R$^6$ obige Bedeutung besitzen, wobei allfällig vorhandene freie Hydroxy-, Amino- und Carboxygruppen in geschützter Form vorliegen,

mit 2 Mol-Aequivalenten Chloracetylisocyanat umsetzt, worauf man allfällig vorhandene Schutzgruppen abspaltet und eine erhaltene Verbindung der Formel I erwünschtenfalls in ein pharmazeutisch annehmbares Salz überführt.

Bei den obigen Verfahren müssen die in den Ausgangsstoffen allfällig vorhandenen reaktionsfähigen freien Hydroxy-, Amino- und Carboxygruppen durch Schutzgruppen blockiert sein. Diese Fälle sind für den Fachmann ohne weiteres erkennbar, und auch die Auswahl der jeweils geeigneten Schutzgruppen bereitet ihm keine Schwierigkeiten. Es kommen insbesondere die in der Peptid-Chemie üblicherweise verwendeten Schutzgruppen für die vorliegenden Zwecke in Betracht.

Eine besonders geeignete Aminoschutzgruppe ist die t-Butoxycarbonylgruppe, welche beispielsweise durch Behandeln mit Trifluoressigsäure, verdünnte HCl/Dioxan oder Natriumjodid/Trimethylchlorsilan/Acetonitril leicht abgespalten werden kann.

Eine besonders geeignete Hydroxyschutzgruppe ist die Benzylgruppe, welche beispielsweise durch Reduktion mit elementarem Wasserstoff an einem geeigneten Katalysator (z.B. Palladiumkohle) leicht abgespalten werden kann. Hier für geeignete Lösungsmittel sind beispielsweise niedere Alkohole, wie Aethanol, und Dimethylformamid.

Eine besonders geeignete Carboxyschutzgruppe ist die p-Nitrobenzylgruppe, welche ebenfalls durch Reduktion mit elementarem Wasserstoff in Gegenwart eines geeigneten Katalysators (z.B. Palladiumkohle) abgespalten werden kann. Auch hierfür eignen sich als Lösungsmittel niedere Alkohole, wie Aethanol, und Dimethylformamid.

Gemäss Verfahrensvariante a) können die Verbindungen der Formel I hergestellt werden, indem man eine Verbindung der Formel II gegebenenfalls in Gegenwart einer Base mit einer Verbindung der Formel III umsetzt. Als Verbindung der Formel III verwendet man vorzugsweise Phosgen oder einen Vorläufer davon oder N,N'-Carbonyldiimidazol, wobei die letztgenannte Verbindung ganz besonders bevorzugt wird. Als Base verwendet man vorzugsweise ein tertiäres Amin, wie Triäthylamin, oder ein bicyclisches Amidin, wie 1,8-Diazabicyclo[5.4.0]undec-7-en (DBU) oder 1,5-Diazabicyclo[4.3.0]non-5-en (DBN). Geeignete Lösungsmittel sind beispielsweise offenkettige und cyclische Aether, wie Diäthyläther, t-Butylmethyläther und Tetrahydrofuran (THF). Die Reaktion wird vorzugsweise in einem Temperaturbereich von etwa Raumtemperatur bis etwa 100 °C durchgeführt.

Gemäss Verfahrensvariante b) können die Verbindungen der Formel I hergestellt werden, indem man eine Verbindung der Formel IV in Gegenwart einer Base mit einer Verbindung der Formel V umsetzt. Als Base verwendet man vorzugsweise ein niederes Alkalimetallalkoholat, wie Natrium- oder Kaliummethanolat. Als Lösungsmittel verwendet man vorzugsweise den entsprechenden niederen Alkohol, z.B. Methanol. Diese Reaktion wird vorzugsweise bei Raumtemperatur durchgeführt.

Gemäss Verfahrensvariante c) können Verbindungen der Formel I hergestellt werden, worin R$^7$ Carboxy, R$^8$ Wasserstoff und Y ein Sauerstoffatom bedeuten. Die Umsetzung einer Verbindung der Formel VI mit 2 Mol-Aequivalenten Chloracetylisocyanat wird vorzugsweise in einem inerten organischen Lösungsmittel durchgeführt, wobei z.B. offenkettige und cyclische Aether, wie Diäthyläther, t-Butylmethyläther und

Tetrahydrofuran, für diesen Zweck in Frage kommen. Die Reaktion wird vorzugsweise in einem Temperaturbereich von 0° C bis zur Raumtemperatur durchgeführt.

Pharmazeutisch annehmbare Salze von Verbindungen der Formel I können nach an sich bekannten und jedem Fachmann geläufigen Methoden hergestellt werden. Verbindungen der Formel I, welche eine freie Carboxygruppe besitzen, können beispielsweise durch Behandeln mit einer geeigneten Base in derartige Salze übergeführt werden. Als Beispiele solcher Salze seien die Alkalimetallsalze, wie die Natrium- und Kaliumsalze erwähnt.

Verbindungen der Formel I, welche eine basische Aminogruppe besitzen, können beispielsweise durch Behandeln mit pharmazeutisch annehmbaren Säuren in Säureadditionssalze übergeführt werden. Als Säureadditionssalze kommen dabei sowohl Salze mit anorganischen als auch Salze mit organischen Säuren in Betracht, beispielsweise Hydrochloride, Hydrobromide, Sulfate, Nitrate, Citrate, Tartrate, Acetate, Maleate, Succinate, Methansulfonate, p-Toluolsulfonate und dergleichen.

Die als Ausgangsstoffe verwendeten Verbindungen der allgemeinen Formeln II, IV und VI sind neu und ebenfalls Gegenstand der vorliegenden Erfindung. Diese Ausgangsstoffe können nach an sich bekannten und jedem Fachmann geläufigen Methoden hergestellt werden. Die weiter unten folgenden Beispiele enthalten detaillierte Angaben betreffend die Herstellung dieser Ausgangsstoffe.

Ebenfalls Gegenstand der Erfindung sind die nach den Verfahrensvarianten a), b) und c) erhältlichen Zwischenprodukte entsprechend der Formel I, worin vorhandene freie Hydroxy-, Amino- und/oder Carboxygruppen in geschützter Form vorliegen.

Wie bereits eingangs erwähnt entfalten die erfindungsgemässen Verbindungen der Formel I und ihre pharmazeutisch annehmbaren Salze eine hemmende Wirkung auf die DNA-Gyrase-Aktivität in Bakterien. Sie können daher zur Verhütung oder Bekämpfung von bakteriellen Infektionen verwendet werden.

Die hemmende Wirkung auf die DNA-Gyrase-Aktivität in Bakterien kann beispielsweise mittels der durch R. Otter und N.R. Cozzarelli in Methods in Enzymology, Vol. 100, pp. 171-180 (1983) beschriebenen Supercoiling-Methode bestimmt werden. Die verwendete DNA-Gyrase wurde aus E. coli N4186 und aus der Untereinheit B von E. coli MK47 (Mitsuchi et al., JBL 159, 9199-9201 (1984)) isoliert. Als Substrat wurde relaxierte pUC18 oder pUC19-Plasmid-DNA verwendet. Die in diesem Versuch ermittelten Ergebnisse sind in der nachfolgenden Tabelle zusammengestellt, wobei die Ergebnisse als MNK (maximale nicht-effektive Konzentration) in µg/ml angegeben sind.

## Tabelle

| Produkt aus | Wirkung<br>MNK in µg/ml | Toxizität<br>$DL_{50}$ in mg/kg |
|---|---|---|
| Beispiel 1 | 2 | - |
| Beispiel 2 | 0,45 | 30 (i.v.)<br>>2000 (p.o.) |
| Beispiel 3 | 2 | - |
| Beispiel 4 | 2 | 30 (i.v.)<br>>4000 (p.o.) |
| Beispiel 8 | 1 | - |

Die Verbindungen der Formel I und ihre pharmazeutisch annehmbaren Salze können als Heilmittel, z.B.in Form pharmazeutischer Präparate zur enteralen oder parenteralen Applikation, Verwendung finden. Sie können beispielsweise peroral, z.B.in Form von Tabletten, Lacktabletten, Dragées, Hart- und Weichgelatinekapseln, Lösungen, Emulsionen oder Suspensionen, rektal, z.B. in Form von Suppositorien, oder parenteral, z.B. in Form von Injektionslösungen, verabreicht werden.

Die Herstellung der pharmazeutischen Präparate kann in jedem Fachmann geläufiger Weise dadurch erfolgen, dass man die erfindungsgemässen Produkte, gegebenenfalls in Kombination mit anderen therapeutisch wertvollen Stoffen, zusammen mit geeigneten, nicht-toxischen, inerten, therapeutisch verträglichen festen oder flüssigen Trägermaterialien und gegebenenfalls den üblichen pharmazeutischen Hilfsstoffen in eine galenische Darreichungsform bringt.

Als Trägermaterialien eignen sich sowohl anorganische als auch organische Trägermaterialien. So kann man für Tabletten, Lacktabletten, Dragees und Hartgelatinekapseln beispielsweise Lactose, Maisstärke oder Derivate davon, Talk, Stearinsäure oder deren Salze als Trägermaterialien verwenden. Für Weichgelatinekapseln eignen sich als Trägermaterialien beispielsweise pflanzliche Oele, Wachse, Fette und halbfeste und flüssige Polyole (je nach Beschaffenheit des Wirkstoffes sind jedoch bei Weichgelatinekapseln keine Träger erforderlich). Zur Herstellung von Lösungen und Sirupen eignen sich als Trägermaterialien beispielsweise Wasser, Polyole, Saccharose, Invertzucker und Glucose. Für Injektionslösungen eignen sich als Trägermaterialien beispielsweise Wasser, Alkohole, Polyole, Glycerin und pflanzliche Oele. Für Suppositorien eignen sich als Trägermaterialien beispielsweise natürliche oder gehärtete Oele, Wachse, Fette und halbflüssige oder flüssige Polyole.

Als pharmazeutische Hilfsstoffe kommen die üblichen Stabilisierungs-, Konservierungs-, Netz- und Emulgiermittel, Mittel zur geschmacklichen Verbesserung, Salze zur Veränderung des osmotischen Drukkes, Puffersubstanzen, Lösungsvermittler, Färbe- und Ueberzugsmittel und Antioxidantien in Frage.

Die Dosierung der Verbindungen der Formel I kann, abhängig von der zu bekämpfenden bakteriellen Infektion, dem Alter und dem individuellen Zustand des Patienten und von der Applikationsweise, innerhalb weiter Grenzen variieren und ist natürlich in jedem einzelnen Fall den individuellen Gegebenheiten anzupassen. Für den erwachsenen Patienten kommt eine tägliche Dosis von etwa 0,05 g bis etwa 4 g, insbesondere etwa 0,1 g bis etwa 2 g in Betracht. Je nach Dosierung ist es dabei zweckmässig, die Tagesdosis in mehreren Dosierungseinheiten zu verabreichen.

Die pharmazeutischen Präparate enthalten zweckmässigerweise etwa 25-2000 mg, vorzugsweise 50-1000 mg eines erfindungsgemässen Produktes.

Die nachfolgenden Beispiele dienen der näheren Erläuterung der vorliegenden Erfindung. Sie sollen deren Umfang jedoch in keiner Weise beschränken. Sämtliche Temperaturen sind in Celsiusgraden angegeben.

Beispiel 1

a) Eine Suspension von Lithiumaluminiumhydrid (44,1 g, 1,16 mMol) in absolutem Aether (700 ml) wird tropfenweise mit einer Lösung von 3′-Chlor-4′-fluoracetanilid (BE Patentschrift Nr. 891537); 109 g, 0,576 Mol) in absolutem THF (350 ml) versetzt. Die erhaltene Suspension wird während 2 Stunden gerührt, dann auf 0° abgekühlt und mit einer Seignettesalzlösung (350 ml) versetzt. Die erhaltenen Kristalle werden abfiltriert und mit Aether gewaschen. Die organische Lösung wird über Magnesiumsulfat getrocknet, und das Lösungsmittel wird unter vermindertem Druck abdestilliert. Man erhält 97,5 g (97,5%) 3-Chlor-N-äthyl-4-fluoranilin als amorphes Material.

b) Eine Lösung von 3-Chlor-N-äthyl-4-fluoranilin (97,4 g, 0.56 mMol) in Eisessig (225 ml) wird bei 5° mit Acetanhydrid (105 ml, 1,12 Mol) versetzt. Die Lösung wird während einer Stunde gerührt, dann auf Eis/Wasser gegossen (250 ml) und mit Essigester extrahiert (2x 200 ml). Die vereinigten Phasen werden nacheinander mit Wasser (100 ml), 2N Natronlauge (100 ml), gesättigter Natriumbicarbonatlösung (100 ml), Wasser (10 ml) und 10-proz. Kochsalzlösung (100 ml) gewaschen. Die Lösung wird über Magnesiumsulfat getrocknet und eingedampft. Der Rückstand wird aus n-Hexan umkristallisiert. Man erhält 102 g (84,2%) 3′-Chlor-N-äthyl-4′-fluoracetanilid.

| | | | | |
|---|---|---|---|---|
| Mikroanalyse $C_{10}H_{11}ClFNO$: | Ber.: | C 55,70 | H 5,14 | N 6,50 |
| | Gef.: | 55,04 | 5,43 | 6,57 |

c) 3′-Chlor-N-äthyl-4′-fluoracetanilid (101 g, 0,468 mMol) wird in konz. Schwefelsäure (300 ml) gelöst. Eine Lösung von Kaliumnitrat (57 g, 0,564 Mol) in konz. Schwefelsäure (220 ml) wird bei 5° dazugetropft. Die erhaltene Lösung wird über Nacht gerührt, dann auf Eis/Wasser gegossen und mit Essigester extrahiert (2x 200 ml). Die organische Phase wird nacheinander mit Wasser (200 ml), gesättigter Natriumbicarbonatlösung (100 ml) und gesättigter Kochsalzlösung (100 ml) gewaschen. Die organische Phase wird mit Aktivkohle behandelt, filtriert und über Magnesiumsulfat getrocknet. Das Lösungsmittel wird abdestilliert, und der Rückstand wird aus Aether/n-Hexan umkristallisiert. Man erhält 74,8 g (61%) 5′-chlor-N-äthyl-4′-fluor-2′-nitroacetanilid mit einem Smp. von 68°.

| Mikroanalyse $C_{10}H_{10}FClN_2O_3$: | Ber.: | C 46,08 | H 3,87 | N 10,75 |
|---|---|---|---|---|
| | Gef.: | 45,99 | 3,92 | 10,82 |

d) 5′-Chlor-N-äthyl-4′-fluor-2′-nitroacetanilid (74 g, 0.284 mMol) wird mit N-Methylpiperazin (126 ml, 1,13 Mol) versetzt, und die erhaltene Lösung wird während 2 Stunden bei 600 gerührt. Das Reaktionsgemisch wird eingedampft, der Rückstand wird in Essigester gelöst (250 ml) und nacheinander mit Wasser (3x 100 ml) und 10-proz. Kochsalzlösung (100 ml) gewaschen. Die organische Lösung wird über Magnesiumsulfat getrocknet und eingedampft. Der Rückstand wird aus Essigester/n-Hexan umkristallisiert. Man erhält 72.3 g (78,5%) N-Aethyl-4′-fluor-5′-(4-methyl-1-piperazinyl) -2′-nitroacetanilid mit einem Smp. von 117-119°.

| Mikroanalyse $C_{15}H_{21}FN_4O_3$: | Ber.: | C 55,50 | H 6,53 | N 17,27 |
|---|---|---|---|---|
| | Gef.: | 55,47 | 6,73 | 17,14 |

e) Eine Lösung von N-Aethyl-4′-fluor-5′-(4-methyl-1-piperazinyl) -2′-nitroacetanilid (20 g, 61 mMol) in Methanol (160 ml) wird mit einer wässrigen Kaliumhydroxidlösung (34,6 g, 616 mMol) versetzt. Die Lösung wird während 3 Stunden auf 800 erhitzt und danach auf Raumtemperatur abgekühlt. Die erhaltenen Kristalle werden abfiltriert und mit Wasser gewaschen. Man erhält 13,7 g (78,7%) 1-(5-(Aethylamino)-2-fluor-4-nitrophenyl)-4-methylpiperazin mit einem Smp. von 149-150°.

| Mikroanalyse $C_{13}H_{19}FN_4O_2$: | Ber.: | C 55,31 | H 6,78 | N 19,85 |
|---|---|---|---|---|
| | Gef.: | 55,16 | 6,95 | 19,81 |

f) Eine Lösung von 1-(5-(Aethylamino)-2-fluor-4-nitrophenyl)-4-methylpiperazin (10 g) in THF (200 ml) wird über 5-proz. Pd/C unter Wasserstoff hydriert. Am Ende der Reduktion wird der Katalysator abfiltriert, das Filtrat wird direkt mit einer Lösung von Natriumcyanat (230 mg) in Wasser (50 ml) versetzt, und der pH wird mit konz. HCl (2,5 ml) auf 3,5 eingestellt. Nach zweistündigem Rühren unter Argon wird der pH mit 4N NaOH auf 8 eingestellt, und die Lösung wird unter vermindertem Druck eingedampft. Der Rückstand wird mit Methanol/Essigester (1:4) extrahiert. Die Lösung wird filtriert, mit Aktivkohle entfärbt und im Vakuum eingedampft. Der Rückstand wird in warmem Essigester aufgenommen. Nach Abkühlen werden die Kristalle abfiltriert und getrocknet. Man erhält 0,63 g (60%) 1-[2-(N-Aethylamino)-5-fluor-4-(4-methyl-1-piperazinyl]phenyl-harnstoff.

| Mikroanalyse $C_{14}H_{22}FN_5O$: | Ber.: | C 56,93 | H 7,51 | N 23,71 |
|---|---|---|---|---|
| | Gef.: | 57,16 | 7,64 | 23,80 |

g) Eine Lösung von 1-[2-(N-Aethylamino)-5-fluor-4-(4-methyl -1-piperazinyl]phenyl-harnstoff (640 mg) in Methanol wird mit einer frisch hergestellten methanolischen Lösung von Natriummethylat (aus 150 mg Natrium) versetzt und für kurze Zeit unter Rückfluss erhitzt. Nach dem Abkühlen wird die Lösung mit Malonsäurediäthylester versetzt und während 6 Stunden gerührt. Die erhaltene Lösung wird auf ein Gemisch von Eis und 2N HCl (50 ml) gegossen. Das Methanol wird abgedampft, und die wässrige Phase wird mit Essigester (3x 100 ml) und Methylenchlorid (2x 100 ml) extrahiert. Nach Trocknen über Magnesiumsulfat wird das Lösungsmittelgemisch eingedampft, und der Rückstand wird in wenig kaltem Essigester aufgeschlämmt und abfiltriert. Man erhält 130 mg (17%) 5-Aethyl-8-fluor-7-(4-methyl -1-piperazinyl)pyrimido[1,6-a]benzimidazol-1,3(2H,5H)-dion.
MS: 345(M)

| Mikroanalyse (Hydrochlorid) $C_{17}H_{21}ClFN_5O_2$: | | | |
|---|---|---|---|
| Ber.: | C 53,47 | H 5,54 | N 18,34 |
| Gef.: | 52,97 | 5,52 | 18,12 |

## Beispiel 2

a) 1-Chlor-2,5-difluor-4-nitrobenzol (3,69 g, 19 mMol) wird bei 0° mit Diäthylamin (2,3 g, 23 mMol) und Cyclopropylamin (1,3 g, 23 mMol) versetzt. Nach 1 Stunde bei 0° wird die erhaltene Suspension während 16 Stunden bei 25° gerührt. Das Reaktionsgemisch wird in Wasser (150 ml) aufgenommen und mit Essigester extrahiert. Die organische Phase wird mit 10-proz. Kochsalzlösung gewaschen und über Magnesiumsulfat getrocknet. Nach Abdestillieren des Lösungsmittels wird der Rückstand aus Aethanol (50 ml) umkristallisiert. Man erhält 3,5 g (79%) 5-Chlor-N-cyclopropyl-4-fluor-2-nitroanilin mit einem Smp. von 73,5-75,5°.

| Mikroanalyse $C_9H_8ClFN_2O_2$: | Ber.: | C 46,87 | H 3,50 | N 12,15 |
|---|---|---|---|---|
| | Gef.: | 46,59 | 3,64 | 12,15 |

b) 5-Chlor-N-cyclopropyl-4-fluor-2-nitroanilin (3,5 g, 15,2 mMol) werden mit N-Acetylpiperazin (3,89 g, 30,4 mMol) und Triäthylamin (2,3 g 22,8 mMol) unter kräftigem Rühren erwärmt und während 12 Stunden bei 60° gehalten. Die erhaltene Masse wird in Wasser (200 ml) gelöst und mit Essigester extrahiert. Die organische Phase wird mit 10-proz. Kochsalzlösung gewaschen und dann über Magnesiumsulfat getrocknet. Nach Abdestillieren des Lösungsmittels wird der Rückstand aus Aethanol umkristallisiert. Man erhält 4,57 g (93%) 1-Acetyl-4-[5-(cyclopropylamino)-2-fluor -4-nitrophenyl]piperazin mit einem Smp. von 142-143°.

| Mikroanalyse $C_{15}H_{19}FN_4O_3$: | Ber.: | C 55,89 | H 5,94 | N 17,38 |
|---|---|---|---|---|
| | Gef.: | 55,66 | 5,94 | 17,35 |

c) 1-Acetyl-4-[5-(cyclopropylamino)-2-fluor-4-nitrophenyl]piperazin (17 g, 53 mMol) wird in Methanol (250 ml) gelöst, mit Kaliumhydroxid (29,6 g, 527 mMol) versetzt und während 3 Stunden unter Rückfluss erhitzt. Das Methanol wird abdestilliert, der Rückstand wird in Essigester gelöst und die organische Phase wird nacheinander mit Wasser und Kochsalzlösung gewaschen und über Magnesiumsulfat getrocknet. Nach Abdestillieren des Lösungsmittels wird der Rückstand in Dioxan (100 ml) gelöst und mit di-tert-Butyl-dicarbonat (13,8 g, 63,24 mMol) und einer wässrigen Lösung von Natriumhydrogencarbonat (6,6 g, 79 mMol, in 60 ml Wasser) versetzt. Nach Rühren während 20 Stunden wird die erhaltene Suspension mit Wasser verdünnt. Die Kristalle werden abfiltriert, mit Wasser gewaschen und aus Aethanol umkristallisiert. Man erhält 18,2 g (91%) tert-Butyl-4-[5-(cyclopropylamino)-2-fluor-4-nitrophenyl]-1-piperazincarboxylat mit einem Smp. von 159-160°.

| Mikroanalyse $C_{18}H_{25}FN_4O_4$: | Ber.: | C 56,83 | H 6,62 | N 14,73 |
|---|---|---|---|---|
| | Gef.: | 56,92 | 6,70 | 14,89 |

d) tert-Butyl-4-[5-(cyclopropylamino)-2-fluor-4-nitrophenyl] -1-piperazincarboxylat (17,21 g, 45,2 mMol) wird in Methanol (500 ml) gelöst und mit 5-Proz. Pd/C (500 mg) unter Wasserstoff bei Normaldruck hydriert. Die Wasserstoffaufnahme beträgt 3,04 1. Die Palladiumkohle wird unter Inertgas abfiltriert, und das Methanol wird unter vermindertem Druck abdestilliert. Der Rückstand wird im Hochvakuum getrocknet, dann in DMF (150 ml) gelöst und mit 3-Aethoxy-3-iminopropansäureäthylester-Hydrochlorid (17,3 g, 88,4 mMol) versetzt. Die erhaltene Lösung wird während 2 Stunden auf 50° erwärmt. Das DMF wird

abdestilliert, der Rückstand wird in Wasser gelöst, und der pH wird mit gesättigter Natriumbicarbonatlösung auf 8,0 eingestellt. Die Lösung wird mit Essigester extrahiert, und die organische Phase wird nacheinander mit Wasser, 10-proz. Kochsalzlösung gewaschen und dann über Magnesiumsulfat getrocknet. Die Lösung wird unter vermindertem Druck eingedampft, und der Rückstand wird an Kieselgel chromatographiert (Laufmittel: Essigester). Das Produkt wird dann aus Essigester/Hexan umkristallisiert. Man erhält 15,5 g (78%) Aethyl 6-[4-(tert-butoxycarbonyl)-1-piperazinyl]-1-cyclopropyl -5-fluor-2-benzimidazolacetat mit einem Smp. von 152-153°.

| | | | | |
|---|---|---|---|---|
| Mikroanalyse $C_{23}H_{31}FN_4O_4$: | Ber.:<br>Gef.: | C 61,87<br>62,13 | H 7,00<br>7,17 | N 12,55<br>12,68 |

e) Aethyl 6-[4-(tert-butoxycarbonyl)-1-piperazinyl]-1-cyclopropyl -5-fluor-2-benzimidazolacetat (4,5 g, 10 mMol) wird in Aethanol (100 ml) gelöst und mit Ammoniumchlorid (5,35 g, 100 mMol) und 25-proz. Ammoniumhydroxidlösung (50 ml) versetzt. Die erhaltene Suspension wird bei 50° während 18 Stunden gerührt und danach unter vermindertem Druck eingedampft. Der Rückstand wird in Essigester (150 ml) suspendiert, und das ungelöste Ammoniumchlorid wird abfiltriert. Das Filtrat wird auf ein Volumen von 50 ml konzentriert und dann an Kieselgel chromatographiert (Laufmittel: Essigester/Methanol 9:1). Das Produkt wird aus Essigester/n-Hexan umkristallisiert. Man erhält 2,22 g (53%) tert-Butyl 4-[2-(carbamoylmethyl)-1-cyclopropyl-5-fluor -6-benzimidazolyl]-1-piperazincarboxylat mit einem Smp. von 219-220°.

| | | | | |
|---|---|---|---|---|
| Mikroanalyse $C_{21}H_{28}FN_5O_3$: | Ber.:<br>Gef.: | C 60,42<br>60,26 | H 6,76<br>6,88 | N 16,78<br>16,31 |

f) tert-Butyl 4-[2-(carbamoylmethyl)-1-cyclopropyl-5-fluor -6-benzimidazolyl]-1-piperazincarboxylat (980 mg, 2,53 mMol) wird in THF (15 ml) suspendiert und mit 1,1'-Carbonyldiimidazol (760 mg, 2 mMol) und 1,8-Diazabicyclo[5.4.0]undec-7-en (0,2 ml) versetzt. Das Reaktionsgemisch wird während 2 Stunden auf 60° erwärmt, wobei weisse Kristalle ausfallen. Die Suspension wird dann auf 0° abgekühlt. Die Kristalle werden abfiltriert und aus Aethanol umkristallisiert. Man erhält 760 mg (73%) tert-Butyl 4-[5-cyclopropyl-8-fluor-1,2,3,5-tetrahydro-1,3 -dioxopyrimido[1,6-a]benzimidazol-7-yl]-1-piperazincarboxylat.

| Mikroanalyse $C_{22}H_{26}FN_5O_4$ mit 0,6 Mol Aethanol: | | | |
|---|---|---|---|
| Ber.:<br>Gef.: | C 59,41<br>59,15 | H 6,67<br>6,33 | N 14,69<br>14,87 |

g) tert-Butyl 4-[5-cyclopropyl-8-fluor-1,2,3,5-tetrahydro-1,3 -dioxopyrimido[1,6-a]benzimidazol-7-yl]-1-piperazincarboxylat (471 mg, 1 mMol) wird in Trifluoressigsäure (2 ml) gelöst. Nach 1 Stunde bei 250 wird die Trifluoressigsäure abdestilliert. Der Rückstand wird in Wasser (10 ml) aufgenommen, mit Natriumhydrogencarbonat (168 mg, 2 mMol) versetzt und während 2 Stunden bei 25° gerührt. Die Suspension wird auf 0° abgekühlt. Die Kristalle werden abfiltriert, nacheinander mit je 5 ml kaltem Wasser und Aethanol gewaschen und aus Aethanol/Wasser (95/5) umkristallisiert. Man erhält 208 mg (60,6%) 5-Cyclopropyl-8-fluor-7-(1-piperazinyl) -pyrimido[1,6-a]benzimidazol-1,3(2H,5H)-dion-trifluoracetat.

| Mikroanalyse $C_{17}H_{18}FN_5O_2 \cdot CF_3COOH$: | | | |
|---|---|---|---|
| Ber.:<br>Gef.: | C 49,89<br>49,71 | H 4,19<br>4,43 | N 15,31<br>15,29 |

Beispiel 3

a) 1-[5-(Aethylamino)-2-fluor-4-nitrophenyl]-4-methylpiperazin (2,8 g, 10 mMol) wird in Methanol (200 ml) gelöst und mit 5-proz. Palladiumkohle unter Wasserstoff bei Normaldruck hydriert. Die Palladiumkohle wird abfiltriert, und das Filtrat wird unter vermindertem Druck eingedampft. Der Rückstand wird im Hochvakuum getrocknet, in Diäthylglykoläthyläther (10 ml) gelöst und dann mit Cyanessigsäureäthylester (2,26 g, 20 mMol) versetzt. Die Lösung wird während 4 Stunden auf 160° erwärmt. Das Lösungsmittel wird dann abdestilliert, und der Rückstand wird in einem Essigester/Aether-Gemisch (100 ml, 1:2) aufgenommen. Die erhaltenen Kristalle werden abfiltriert, in einem Essigester/Methanol-Gemisch (6:4) gelöst und an Kieselgel chromatographiert (Laufmittel: Essigester/Methanol 6:4). Das Produkt wird aus einem Essig ester/Acetonitril-Gemisch (9:1) umkristallisiert. Man erhält 1,17 g (39%) 1-Aethyl-5-fluor-6-(4-methyl-1-piperazinyl) -2-benzimidazolacetonitril mit einem Smp. von 204-206°.

| Mikroanalyse $C_{16}H_{20}FN$: | Ber.: | C 63,77 | H 6,69 | N 23,24 |
|---|---|---|---|---|
| | Gef.: | 63,65 | 6,64 | 23,31 |

b) 1-Aethyl-5-fluor-6-[4-methyl-1-piperazinyl)-2-benzimidazolacetonitril (834 mg, 2,77 mMol) wird in Pyridin (20 ml) und Triäthylamin (2,8 g, 27,7 mMol) gelöst. Die Lösung wird auf 0° abgekühlt. Anschliessend wird während 30 Minuten Schwefelwasserstoff eingeleitet. Die so erhaltene, tiefgrüne Lösung wird während 2 Stunden auf 45° erwärmt. Das Lösungsmittel wird abdestilliert, und der Rückstand wird im Hochvakuum getrocknet und dann aus Acetonitril kristallisiert. Die Kristalle werden abfiltriert, in einem Essigester/Methanol-Gemisch (10 ml, 1:1) gelöst und dann an Kieselgel chromatographiert (Laufmittel: Essigester/Methanol 6:4). Das Produkt wird aus Essigester umkristallisiert. Man erhält 745 mg (80%) 1-Aethyl-5-fluor-6-[4-methyl-1-piperazinyl] -2-benzimidazolcarbothioamid.

| Mikroanalyse $C_{16}H_{22}FN_5S \cdot 0,3\ H_2O$: | | | |
|---|---|---|---|
| Ber.: | C 56,38 | H 6,68 | N 20,55 |
| Gef.: | 56,32 | 6,76 | 20,41 |

c) 1-Aethyl-5-fluor-6-[4-methyl-1-piperazinyl]-2-benzimidazolcarbothioamid (120 mg, 0,36 mMol) wird in Tetrahydrofuran (2 ml) suspendiert und mit Carbonyldiimidazol (140 mg, 0,86 mMol) versetzt. Die Reaktionslösung wird während 6 Stunden auf 68° erwärmt, wobei weisse Kristalle ausfallen. Die Suspension wird auf 0° abgekühlt. Die Kristalle werden abfiltriert, in Methanol suspendiert und nach einer Stunde erneut abfiltriert, mit Aether gewaschen und im Hochvakuum getrocknet. Man erhält 22 mg (16,9%) 5-Aethyl-9-fluor-3,5-dihydro-8-(4-methyl-1-piperazinyl) -3-thioxopyrimido[1,6-a]benzimidazol-1-(2H)-on.

| Mikroanalyse $C_{17}H_{20}FN_5OS \cdot 0,7\ H_2O$: | | | |
|---|---|---|---|
| Ber.: | C 54,59 | H 5,77 | N 18,72 |
| Gef.: | 54,37 | 5,84 | 18,64 |

Beispiel 4

a) tert-Butyl 4-[2-carbamoylmethyl)1-cyclopropyl-5-fluor -6-benzimidazolyl]-1-piperazincarboxlyat (703 mg, 1,68 mMol) wird mit O-Benzylhydroxylamin-Hydrochlorid (806 mg, 5,05 mMol) in einem Wasser/Aethanol-Gemisch (5 ml, 1:1) während 72 Stunden auf 50° erwärmt. Das Lösungsmittel wird abdestilliert, und der Rückstand wird in Wasser (20 ml) gerührt. Die erhaltenen Kristalle werden abfiltriert, mit Wasser gewaschen und im Hochvakuum getrocknet. Das Produkt wird aus Essigester umkristallisiert. Man erhält 317 mg (36%) tert-Butyl 4-[2-[[(benzyloxy)carbamoyl]methyl]-1-cyclopropyl-5-fluor -6-benzimidazolyl]-1-piperazincarboxylat.

| | | | | |
|---|---|---|---|---|
| Mikroanalyse $C_{28}H_{34}FN_5O_4$: | Ber.: | C 64,23 | H 6,55 | N 13,38 |
| | Gef.: | 64,35 | 6,83 | 13,35 |

b) tert-Butyl 4-[2-([(benzyloxy)carbamoyl]methyl]-1-cyclopropyl-5-fluor -6-benzimidazolyl]-1-piperazincarboxylat (779 mg, 1,18 mMol) wird in Tetrahydrofuran (15 ml) gelöst und mit N,N′-Carbonyldiimidazol (480 mg, 2,96 mMol) und 1,8-Diazabicyclo[5.4.0]undec-7-en (225 mg, 1,48 mMol) versetzt. Die erhaltene Suspension wird auf Wasser (200 ml) gegossen und dann mit Essigester extrahiert. Die organische Phase wird nacheinander mit Wasser und 10-proz. Kochsalzlösung gewaschen und über Magnesiumsulfat getrocknet. Das Lösungsmittel wird unter vermindertem Druck abgedampft, und der Rückstand wird an Kieselgel chromatographiert (Laufmittel: Essigester/n-Hexan 8:2). Das Produkt wird aus Essigester/n-Hexan kristallisiert. Man erhält 42 mg (5%) tert-Butyl 4-[2-(benzyloxy)-5-cyclopropyl-8-fluor-1,2,3,5-tetrahydro -1,3-dioxopyrimido[1,6-a]benzimidazol-7-yl] -1-piperazincarboxylat.

| | | | | |
|---|---|---|---|---|
| Mikroanalyse $C_{29}H_{32}FN_5O_5$: | Ber.: | C 63,38 | H 5,87 | N 12,74 |
| | Gef.: | 62,78 | 5,69 | 12,64 |

c) tert-Butyl 4-[2-(benzyloxy)-5-cyclopropyl-8-fluor-1,2,3,5-tetrahydro -1,3-dioxopyrimido[1,6-a]-benzimidazol-7-yl] -1-piperazincarboxylat (981 mg, 1,87 mMol) wird in Aethanol (250 ml) gelöst und mit 5-proz. Palladiumkohle (200 mg) unter Wasserstoff bei Normaldruck hydriert. Die Palladiumkohle wird abfiltriert und mit Dimethylformamid (100 ml) bei 100° gewaschen. Die vereinigten Filtrat werden unter vermindertem Druck eingedampft. Der Rückstand wird aus Dimethylformamid umkristallisiert. Man erhält 619 mg (72%) tert-Butyl 4-[5-cyclopropyl-8-fluor-1,2,3,5-tetrahydro-2-hydroxy-1,3 -dioxopyrimido[1,6-a]-benzimidazol-7-yl]-1-piperazincarboxylat.

| | | | | |
|---|---|---|---|---|
| Mikroanalyse $C_{22}H_{26}FN_5O_5$: | Ber.: | C 57,51 | H 5,70 | N 15,24 |
| | Gef.: | 57,27 | 5,65 | 15,38 |

d) tert-Butyl 4-[5-cyclopropyl-8-fluor-1,2,3,5-tetrahydro-2-hydroxy-1,3 -dioxopyrimido[1,6-a]benzimidazol-7-yl]-1-piperazincarboxylat (350 mg, 0,76 mMol) wird mit einer 2,5N Chlorwasserstoffsäure-Lösung in Dioxan (4 ml) versetzt, wobei sich sofort ein weisser Niederschlag bildet. Die Suspension wird während 18 Stunden bei Raumtemperatur (25°) gerührt. Die ausgefallenen Kristalle werden abfiltriert und in Wasser (10 ml) gelöst. Die Lösung wird mit Aktivkohle behandelt, dann filtriert und auf 0° abgekühlt. Die ausgefallenen Kristalle werden abfiltriert, mit Aethanol und Aether gewaschen und im Hochvakuum getrocknet. Man erhält 176 mg (58%) 5-Cyclopropyl-8-fluor-2-hydroxy-7-(1-piperazinyl) -pyrimido[1,6-a]-benzimidazol-1,3(2H,5H)-dion-Hydrochlorid.

| | | | | |
|---|---|---|---|---|
| Mikroanalyse $C_{17}H_{19}FN_5O_3$: | Ber.: | C 51,59 | H 4,84 | N 17,69 |
| | Gef.: | 51,27 | 5,11 | 17,54 |

Beispiel 5

a) 5-Chlor-N-cyclopropyl-4-fluor-2-nitroanilin (460 mg, 2 mMol) wird mit L-Prolin-tert-Butylester (342 mg, 2 mMol) und 1,8-Diazabicyclo[5.4.0]undec-7-en (304 mg, 2 mMol) während 40 Stunden auf 80° erwärmt. Das Reaktionsgemisch wird dann in einem Wasser/Essigester-Gemisch (1:1, 100 ml) gelöst. Die Essigesterphase wird mit 10-proz. Kochsalzlösung gewaschen, über Magnesiumsulfat getrocknet und unter vermindertem Druck eingedampft. Der Rückstand wird an Kieselgel chromatographiert (Laufmittel: Hexan/Essigester 8:2). Das Produkt wird aus Aether/n-Hexan kristallisiert. Man erhält 94 mg (13%) tert-Butyl rac-1-[5-(cyclopropylamino)-2-fluor-4-nitrophenyl]pyrrol -2-carboxylat mit einem Smp. von 142-143°.

| Mikroanalyse $C_{18}H_{24}FN_3O_4$: | Ber.: | C 59,17 | H 6,62 | N 11,50 |
|---|---|---|---|---|
| | Gef.: | 59,23 | 6,62 | 11,52 |

b) In Analogie zu Beispiel 2d) erhält man aus tert-Butyl rac-1-[5-(cyclopropylamino)-2-fluor-4-nitrophenyl]pyrrol -2-carboxylat in eine Ausbeute von 56% das Aethyl rac-6-[2-(tert-butoxycarbonyl)-1-pyrrolidinyl]-1-cyclopropyl -5-fluor-2-benzimidazolacetat mit einem Smp. von 93-96°.

| Mikroanalyse $C_{23}H_{30}FN_3O_4$: | Ber.: | C 64,02 | H 7,01 | N 9,74 |
|---|---|---|---|---|
| | Gef.: | 64,47 | 7,16 | 9,77 |

c) In Analogie zu Beispiel 2e) erhält man aus Aethyl rac-6-[2-(tert-butoxycarbonyl)-1-pyrrolidinyl]-1-cyclopropyl -5-fluor-2-benzimidazolacetat in einer Ausbeute von 34% das tert-Butyl rac-1-[2-(carbamoylmethyl)-1-cyclopropyl-5-fluor -6-benzimidazolyl]-2-pyrrolidincarboxylat mit einem Smp. von 176-178°.

| Mikroanalyse $C_{21}H_{27}FN_4O_3$: | Ber.: | C 62,67 | H 6,76 | N 13,92 |
|---|---|---|---|---|
| | Gef.: | 62,40 | 6,86 | 13,60 |

d) In Analogie zu Beispiel 2f) erhält man aus tert-Butyl rac-1-[2-(carbamoylmethyl)-1-cyclopropyl-5-fluor -6-benzimi dazolyl]-2-pyrrolidincarboxylat in einer Ausbeute von 60% das tert-Butyl rac-1-[5-cyclopropyl-8-fluor-2,3-dihydro-1,3 -dioxopyrimido[1,6-a]benzimidazol-7(1H)-yl] -2-pyrrolidincarboxylat mit einem Smp. von 226-227°.

| Mikroanalyse $C_{22}H_{25}FN_4O_4 \cdot 0{,}25$ THF | | | |
|---|---|---|---|
| Ber.: | C 61,87 | H 6,10 | N 12,55 |
| Gef.: | 61,77 | 6,38 | 12,62 |

## Beispiel 6

a) tert-Butyl 4-[2-(benzyloxy)-5-cyclopropyl-8-fluor-1,2,3,5-tetrahydro -1,3-dioxopyrimido[1,6-a]-benzimidazol-7-yl] -1-piperazincarboxylat (580 mg, 1,05 mMol) wird mit 33-proz. Bromwasserstoffsäure in Eisessig (5 ml) versetzt. Nach 18 Stunden bei Raumtemperatur werden die ausgefallenen Kristalle abfiltriert, mit Eisessig gewaschen und aus Methanol umkristallisiert. Die Kristalle werden dann in einem Wasser/Aethanol-Gemisch (2:1, 50 ml) gelöst. Der pH wird mittels 1H Natronlauge auf 8 eingestellt, wobei weisse Kristalle ausfallen. Die Kristalle werden abfiltriert, mit Wasser gewaschen und im Hochvakuum getrocknet. Man erhält 417 mg (88%) 2-(Benzyloxy)-5-cyclopropyl-8-fluor-7 -(1-piperazinyl)-pyrimido[1,6-a]benzimidazol-1,3(2H,5H)-dion.

| Mikroanalyse $C_{24}H_{24}FN_5O_3$: | Ber.: | C 64,13 | H 5,38 | N 15,58 |
|---|---|---|---|---|
| | Gef.: | 63,81 | 5,68 | 15,54 |

b) Z-(L)-Alanin (223 mg, 1 mMol) wird in Dichlormethan (10 ml) gelöst und bei 0° mit Dicyclohexylcarbodiimid (103 mg, 0,5 mMol) versetzt. Nach 30 Minuten werden die Kristalle abfiltriert und mit wenig (30 ml) Dichlormethan gewaschen. Das Filtrat wird mit einer Lösung von 2-(Benzyloxy)-5-cyclopropyl-8-fluor-7 -(1-piperazinyl)pyrimido[1,6-a]benzimidazol-1,3(2H,5H)-dion (112 mg, 0,25 mMol) in DMF (5 ml) versetzt. Nach 2 Stunden wird das Lösungsmittel abdestilliert. Der Rückstand wird aus Methanol umkristallisiert. Man erhält 142 mg (86%) Benzyl [(S)-1-[[4-[2-(benzyloxy)-5-cyclopropyl-8-fluor-1,2,3,5 -tetrahydro-1,3-dioxo pyrimido[1,6-a]benzimidazol-7-yl] -1-piperazinyl]carbonyl]äthyl]carbamat.
MS: 653(M).

c) Benzyl [(S)-1-[[4-[2-(benzyloxy)-5-cyclopropyl-8-fluor-1,2,3,5 -tetrahydro-1,3-dioxopyrimido[1,6-a]-benzimidazol-7-yl] -1-piperazinyl]carbonyl]äthyl]carbamat (127 mg, 0,194 mMol) wird in DMF (15 ml) gelöst und über 5-proz. Pd/C hydriert. Nach Beendigung der Reduktion wird der Katalysator abfiltriert, und das Filtrat wird eingedampft. Der Rückstand wird in Methanol (50 ml) aufgenommen und mit Aktivkohle behandelt. Nach Abfiltrieren der Kohle, wird das Filtrat auf ein Volumen von 25 ml konzentriert. Die ausgefallenen Kristalle werden abfiltriert. Man erhält 62 mg (84%) 7-(4-L-Alanyl-1-piperazinyl)-5-cyclopropyl-8-fluor -2-hydroxypyrimido[1,6-a]benzimidazol-1,3(2H,5H)-dion.

| Mikroanalyse $C_{20}H_{23}FN_6O_4 \cdot 0,25\ CH_3OH$: | | | |
|---|---|---|---|
| Ber.:<br>Gef.: | C 55,47<br>55,15 | H 5,52<br>5,76 | N 19,17<br>19,18 |

## Beispiel 7

a) Eine Lösung von Aethyl rac-6-[2-(tert-butoxycarbonyl)-1-pyrrolidinyl]-1-cyclopropyl -5-fluor-2-benzimidazolacetat (1,647 g, 3,82 mMol) in Aethanol (15 ml) wird mit einer 1N äthanolischen Lösung von Hydroxylamin (9,54 ml, 9,54 mMol) und mit einer 1N äthanolischen Natriummethylatlösung (1,91 ml, 1,91 mMol) versetzt. Nach einer Stunde wird das Lösungsmittel abdestilliert, und der Rückstand wird in Wasser gelöst. Der pH wird mit 1N Salzsäure auf 5,5 eingestellt. Diese Lösung wird mit Essigester extrahiert, und die organische Phase wird mit Wasser gewaschen, getrocknet (Magnesiumsulfat) und eingeengt. Der Rückstand wird aus Essigester umkristallisiert. Die erhaltenen Kristalle werden in Tetrahydrofuran (30 ml) gelöst und mit 1,8-Diazabicyclo[5.4.0]undec-7-en (377 mg, 2,5 mMol) und Benzylbromid (462 mg, 2,7 mMol) versetzt. Nach 4 Stunden wird die Reaktionslösung mit Essigester (150 ml) verdünnt und mit Wasser (100 ml) und anschliessend mit 10-proz. Kochsalzlösung (100 ml) gewaschen. Die organische Phase wird getrocknet (Magnesiumsulfat) und eingeengt. Der Rückstand wird aus Aether/n-Hexan umkristallisiert. Man erhält 643 mg (33%) 1-[2-[[(Benzyloxy)carbamoyl]methyl]-1-cyclopropyl-5-fluor -6-benzimidazolyl]-L-prolin-tert-butylester.

| | | | | |
|---|---|---|---|---|
| Mikroanalyse $C_{28}H_{33}FN_4O_4$: | Ber.:<br>Gef.: | C 66,13<br>65,99 | H 6,54<br>6,86 | N 11,01<br>11,14 |

b) In Analogie zu Beispiel 4b) erhält man aus 1-[2-[[(Benzyloxy)carbamoyl]methyl]-1-cyclopropyl-5-fluor -6-benzimidazolyl]-L-prolin-tert-butylester in einer Ausbeute von 78% den 1-[2-(Benzyloxy)-5-cyclopropyl-8-fluor-1,2,3,5-tetrahydro -1,3-dioxopyrimido[1,6-a]benzimidazol-7-yl]-L-prolin -tert-butylester.
MS: 534(M).

c) In Analogie zu Beispiel 4c) erhält man aus 1-[2-(Benzyloxy)-5-cyclopropyl-8-fluor-1,2,3,5-tetrahydro -1,3-dioxopyrimidol[1,6-a]benzimidazol-7-yl]-L-prolin -tert-butylester in einer Ausbeute von 81% den 1-[5-Cyclopropyl-8-fluor-1,2,3,5-tetrahydro-2-hydroxy-1,3 -dioxopyrimido[1,6-a]benzimidazol-7-yl]-L-prolin -tert-butylester.

| | | | | |
|---|---|---|---|---|
| Mikroanalyse $C_{22}H_{25}FN_4O_5$: | Ber.:<br>Gef.: | C 59,45<br>59,03 | H 5,67<br>5,54 | N 12,61<br>12,51 |

## Beispiel 8

a) In Analogie zu Beispiel 1d) erhält man aus N-Acetylpiperazin und 5′-Chlor-N-äthyl-4′-fluor-2′-nitroacetanilid in einer Ausbeute von 76% das N-Aethyl-4′-fluor-5′-(4-acetyl-1-piperazinyl) -2′-nitroacetanilid mit einem Smp. von 129-131°.

| Mikroanalyse $C_{16}H_{21}FN_4O_4$: | Ber.:<br>Gef.: | C 54,54<br>54,33 | H 6,01<br>6,18 | N 15,90<br>15,80 |
|---|---|---|---|---|

b) Eine Lösung von N-Aethyl-4′-fluor-5′-(4-acetyl-1-piperazinyl) -2′-nitroacetanilid (8,4 g, 23,8 mMol) in Methanol (100 ml) wird mit Kalilauge (13,3 g, 238 mMol in 20 ml) versetzt. Die Lösung wird während 3 Stunden unter Rückfluss erhitzt und dann auf Eis gegossen. Die ausgefallenen Kristalle werden abfiltriert (Smp. 129-131°), in Dioxan/Wasser 1:1 (200 ml) suspendiert und mit Di-tert-butyl-dicarbonat (4,44 g, 20,35 mMol) und Natriumbicarbonat (1,71 g, 20,35 mMol) versetzt. Nach 24 Stunden wird das Reaktionsgemisch eingedampft. Der Rückstand wird in Essigester (500 ml) aufgenommen und mit Wasser (500 ml) und 10-proz. Kochsalzlösung gewaschen. Die organische Phase wird über Magnesiumsulfat getrocknet und eingeengt. Der Rückstand wird aus Essigester/n-Hexan kristallisiert. Man erhält 6,33 g (72%) tert-Butyl 4-[5-(äthylamino)-2-fluor-4-nitrophenyl] -1-piperazincarboxylat mit einem Smp. von 155-157°.

c) In Analogie zu Beispiel 2d) erhält man aus tert-Butyl 4-[5-(äthylamino)-2-fluor-4-nitrophenyl] -1-piperazincarboxylat in einer Ausbeute von 67% das Aethyl 6-[4-tert-butoxycarbonyl)-1-piperazinyl]-1-äthyl-5-fluor -2-benzimidazolacetat mit einem Smp. von 131-133°.

| Mikroanalyse $C_{22}H_{31}FN_4O_4$: | Ber.:<br>Gef.: | C 60,81<br>60,73 | H 7,19<br>7,44 | N 12,89<br>12,88 |
|---|---|---|---|---|

d) In Analogie zu Beispiel 2e) erhält man aus Aethyl 6-[4-tert-butoxycarbonyl)-1-piperazinyl]-1-äthyl-5-fluor -2-benzimidazolacetat in einer Ausbeute von 36% das tert-Butyl 4-[2-carbamoylmethyl)-1-äthyl-5-fluor -6-benzimidazolyl]-1-piperazincarboxylat mit einem Smp. von 162-164°.
MS: 405(M).

e) In Analogie zu Beispiel 2f) erhält man aus tert-Butyl 4-[2-(carbamoylmethyl)-1-äthyl-5-fluor -6-benzimidazolyl]-1-piperazincarboxylat in einer Ausbeute von 67% das tert-Butyl 4-(5-äthyl-8-fluor-1,2,3,5-tetrahydro-1,3 -dioxo pyrimido[1,6-a]benzimidazol-7-yl)-1-piperazincarboxylat mit einem Smp. von 270-273°.

| Mikroanalyse $C_{21}H_{26}FN_5O_4$: | Ber.:<br>Gef.: | C 58,46<br>58,18 | H 6,07<br>6,30 | N 16,23<br>16,16 |
|---|---|---|---|---|

f) In Analogie zu Beispiel 2g) erhält man aus tert-Butyl 4-(5-äthyl-8-fluor-1,2,3,5-tetrahydro-1,3 -dioxopyrimido[1,6-a]benzimidazol-7-yl)-1-piperazincarboxylat in einer Ausbeute von 70% das 5-Aethyl-8-fluor-7-(1-piperazinyl) -pyrimido[1,6-a]benzimidazol-1,3(2H,5H)-dion mit einem Smp. von 264-266°.

| Mikroanalyse $C_{16}H_{18}FN_5O_2 \cdot 0{,}2\ CH_2OH$: | | | |
|---|---|---|---|
| Ber.:<br>Gef.: | C 57,61<br>57,44 | H 5,61<br>5,49 | N 20,74<br>20,85 |

Beispiel 9

a) In Analogie zu Beispiel 2d) erhält man aus 1-(5-Aethylamino)-2-fluor-4-nitrophenyl)-4-methylpiperazin in einer Ausbeute von 30% das Aethyl 1-äthyl-5-fluor-6-(4-methyl-1-piperazinyl) -2-benzimidazolacetat mit einem Smp. von 142-144°.

| Mikroanalyse $C_{18}H_{25}FN_4O_2$: | Ber.:<br>Gef.: | C 62,05<br>61,68 | H 7,23<br>7,31 | N 16,08<br>16,09 |
|---|---|---|---|---|

b) In Analogie zu Beispiel 2e) erhält man aus Aethyl 1-äthyl-5-fluor-6-(4-methyl-1-piperazinyl) -2-benzimidazolacetat das 1-Aethyl-5-fluor-6-(4-methyl-1-piperazinyl) -2-benzimidazolacetamid. Aus diesem Zwischenprodukt erhält man in Analogie zu Beispiel 4a)in einer Ausbeute von 35% (über beide Stufen) das 2-[[(Benzyloxy)carbamoyl]methyl]-1-äthyl-5-fluor-6-(4-methyl -1-piperazinyl)-benzimidazol mit einem Smp. von 165-168° (Essigester).

| | | | | |
|---|---|---|---|---|
| Mikroanalyse $C_{23}H_{28}FN_5O_2$: | Ber.: | C 64,92 | H 6,63 | N 16,46 |
| | Gef.: | 64,92 | 6,85 | 16,36 |

c) In Analogie zu Beispiel 4b) erhält man aus 2-[[(Benzyloxy)carbamoyl]methyl]-1-äthyl-5-fluor-6-(4-methyl -1-piperazinyl)benzimidazol in einer Ausbeute von 54% das 2-(Benzyloxy)-5-äthyl-8-fluor-7-(4-methyl -1-piperazinyl)pyrimido[1,6-a]benzimidazol-1,3(2H,5H)-dion mit einem Smp. von 250-252° (Methanol).

| | | | | |
|---|---|---|---|---|
| Mikroanalyse $C_{24}H_{26}FN_5O_3$: | Ber.: | C 63,85 | H 5,80 | N 15,51 |
| | Gef.: | 63,64 | 6,13 | 15,43 |

d) In Analogie zu Beispiel 4c) erhält man aus 2-(Benzyloxy)-5-äthyl-8-fluor-7-(4-methyl -1-piperazinyl)-pyrimido[1,6-a]benzimidazol-1,3(2H,5H)-dion in eine Ausbeute von 53% das 4-Aethyl-8-fluor-2-hydroxy-7-(4-methyl -1-piperazinyl)pyrimido[1,6-a]benzimidazol-1,3(2H,5H)-dion.

| Mikroanalyse $C_{17}H_{20}FN_2O_3 \cdot 0,2$ DMF: | | | |
|---|---|---|---|
| Ber.: | C 56,22 | H 5,74 | N 19,37 |
| Gef.: | 56,05 | 5,90 | 19,40 |

Beispiel 10

a) In Analogie zu Beispiel 2d) erhält man aus 3-Aethoxy-3-iminopropansäure-p-nitrobenzylester-Hydrochlorid und tert-Butyl 4-[5-(cyclopropylamino)-2-fluor-4-nitrophenyl] -1-piperazincarboxylat in einer Ausbeute von 44% das p-Nitrobenzyl 6-[4-(tert-butoxycarbonyl)-1-piperazinyl]-1-cyclopropyl -5-fluor-2-benzimidazolacetat.

| | | | | |
|---|---|---|---|---|
| Mikroanalyse $C_{28}H_{32}FN_5O_6$: | Ber.: | C 60,75 | H 5,83 | N 12,65 |
| | Gef.: | 60,81 | 6,11 | 12,54 |

b) Eine Lösung von p-Nitrobenzyl 6-[4-(tert-Butoxycarbonyl)-1-piperazinyl]-1-cyclopropyl -5-fluor-2-benzimidazolacetat (553 mg, 1 mMol) in THF (50 ml) wird nacheinander bei 0° mit Triäthylamin (300 mg, 3 mMol) und Chloracetylisocyanat (298 mg, 2.5 mMol) versetzt . Nach Rühren während 20 Stunden wird das Lösungsmittel abdestilliert, und der Rückstand wird in Essigester aufgenommen und nacheinander mit Wasser (50 ml) und 10-proz. Kochsalzlösung (50 ml) gewa schen. Die organische Phase wird über Magnesiumsulfat getrocknet, und das Lösungsmittel wird abgedampft. Der Rückstand wird aus Essigester umkristallisiert. Man erhält 373 mg (59%) p-Nitrobenzyl 5-cyclopropyl-8-fluor-1,2,3,4-tetrahydro-1,3-dioxo-7-[4-(tert butoxycarbonyl)-1-piperazinyl]pyrimido[1,6a]benzimidazol -4-carboxylat.

| | | | | |
|---|---|---|---|---|
| Mikroanalyse $C_{30}H_{31}FN_6O_8$: | Ber.: | C 57,87 | H 5,02 | N 13,50 |
| | Gef.: | 57,50 | 5,04 | 13,38 |

c) Eine Lösung von p-Nitrobenzyl 5-cyclopropyl-8-fluor-1,2,3,4-tetrahydro-1,3-dioxo-7-(4-(tert - butoxycarbonyl)-1-piperazinyl)pyrimido[1,6a]benzimidazol -4-carboxylat (1,758 g, 2,82 mMol) in Acetonitril (250 ml) wird nacheinander mit Natriumjodid (846 mg, 5,65 mMol) und Trimethylchlorsilan (1.23 g, 11,28 mMol) versetzt. Nach einer Stunde bei 80° wird die erhaltene Suspension auf 0° abgekühlt. Die Kristalle werden abfiltriert und in einem Aethanol/Wasser-Gemisch (2:8, 250 ml) aufgenommen. Der pH wird mit einer Natriumbicarbonatlösung auf 8,0 eingestellt. Die Suspension wird auf 0° abgekühlt, und die hellgelben Kristalle werden abfiltriert. Der Filterrückstand wird in Aethanol aufgeschlämmt. Die Kristalle werden abfiltriert und mit Aether gewaschen. Man erhält 1,10 g (75%) p-Nitrobenzyl 5-cyclopropyl-8-fluor-1,2,3,4-tetrahydro-1,3-dioxo-7 -(1-piperazinyl)pyrimido[1,6-a]benzimidazol-4-carboxylat.

| | | | | |
|---|---|---|---|---|
| Mikroanalyse $C_{25}H_{23}FN_6O_6$: | Ber.: | C 57,47 | H 4,44 | N 16,08 |
| | Gef.: | 57,13 | 4,48 | 15,97 |

d) Eine Lösung von p-Nitrobenzyl 5-cyclopropyl-8-fluor-1,2,3,4-tetrahydro-1,3-dioxo -7-(1-piperazinyl)-pyrimido[1,6-a]benzimidazol-4-carboxylat (122 mg, 0,234 mMol) in DMF (30 ml) wird über 5-proz. Pd/C hydriert. Nach Beendigung der Hydrierung wird der Katalysator abfiltriert, und das Filtrat wird mit Bleicherde behandelt, filtriert und auf ein Volumen von 5 ml konzentriert. Die ausgefallenen Kristalle werden abfiltriert. Man erhält 62 mg (68%) des Natriumsalzes der 5-Cyclopropyl-8-fluor-1,2,3,4-tetrahydro-1,3-dioxo -7-(1-piperazinyl)pyrimido[1,6-a]benzimidazol-4-carbonsäure.
MS: 343(M-$CO_2$), 44 ($CO_2$).

Die Kristalle werden in DMF (10 ml) aufgenommen und mit einer gesättigten Natriumbicarbonatlösung (1 ml) versetzt. Das Lösungsmittel wird abdestilliert, und der Rückstand wird mit Wasser an einer Reversphase (Säule RPB) chromatographiert. Man erhält 39 mg (40%) des obigen Produktes.
MS: 343 (M-$CO_2$), 44 ($CO_2$).

## Beispiel 11

In Analogie zu Beispiel 9 erhält man aus tert-Butyl 4-[2-(carbamoylmethyl)-1-äthyl-5-fluor-6-benzimidazolyl] -1-piperazincarboxylat (aus Beispiel 8d) nach Abspaltung der tert-Butoxycarbonylgruppe mittels 2,5N HCl/Dioxan (in Analogie zu Beispiel 4d) das 5-Aethyl-8-fluor-2-hydroxy -7-(1-piperazinyl)-pyrimido[1,6-a]benzimidazol -1,3(2H,5H)-dion-Hydrochlorid.

| | | | | |
|---|---|---|---|---|
| Mikroanalyse $C_{16}H_{19}ClFN_5O_3$: | Ber.: | C 50,07 | H 4,99 | N 18,25 |
| | Gef.: | 50,94 | 5,22 | 18,47 |

## Beispiel 12

a) Aethyl-6-[4-(tert-butoxycarbonyl)-1-piperazinyl]-1-cyclopropyl-5-fluor-2-benzimidazolacetat (1,8 g, 4 mmol; aus Beispiel 2d) und tert-Butylcarbazat (21 g, 16 mmol) werden in Pyridin (40 ml) gelöst und 65 Stunden bei 115° unter Argon gerührt. Die Reaktionslösung wird eingeengt und mit 50 ml Aether verrührt. Das ausgefallene Produkt wird abgenutscht und getrocknet. Man erhält tert-Butyl-3-[[6-[4-(tert-butoxycarbonyl)-1-piperazinyl]-1-cyclopropyl-5-fluor-2-benzimidazolyl]acetyl]carbazat. Ausbeute: 0,49 g (23%); Smp. 202-203°.

| | | | | |
|---|---|---|---|---|
| Mikroanalyse $C_{26}H_{37}FN_6O_5$: | Ber.: | C 58,63 | H 7,00 | N 15,78 |
| | Gef.: | 58,44 | 7,15 | 15,66 |

b) tert-Butyl 3-[]6-[4-(tert-butoxycarbonyl)-1-piperazinyl]-1-cyclopropyl-5-fluor-2-benzimidazolyl]acetyl]-

carbazat (0,40 g, 0,76 mmol) wird in THF (15 ml) gelöst und mit 1,1′-Carbonyldiimidazol (0,24 g, 1,5 mmol) und DBU (3 Tropfen) versetzt. Die erhaltene Lösung wird 2 Stunden bei 60° gerührt und danach am Rotationsverdampfer eingeengt. Der Rückstand wird in Essigester gelöst und dann auf Kieselgel chromatographiert (Laufmittel: Essigester/Hexan 4:1). Das Produkt wird aus Essigester/Aether umkristallisiert. Man erhält tert-Butyl 7-[4-(tert-butoxycarbonyl)-1-piperazinyl]-5-cyclopropyl-8-fluor-3,5-dihydro-1,3-dioxopyrimido[1,6-a]benzimidazol-2(1H)-carbamat. Ausbeute: 85 mg (20%); Smp. 219-220°.

| | | | | |
|---|---|---|---|---|
| Mikroanalyse $C_{27}H_{35}FN_6O_6$: | Ber.: | C 58,05 | H 6,32 | N 15,04 |
| | Gef.: | 57,87 | 6,36 | 14,69 |

c) tert-Butyl 7-[4-(tert-butoxycarbonyl)-1-piperazinyl]-5-cyclopropyl-8-fluor-3,5-dihydro-1,3-dioxopyrimido-[1,6-a]benzimidazol-2(1H)-carbamat (0,145 g, 0.26 mmol) wird in 1 ml Trifluoressigsäure gelöst und 2 Stunden bei Raumtemperatur gerührt. Die Reaktionslösung wird eingeengt, mit 1 ml $H_2O$ versetzt, mit gesättigter wässriger $NaHCO_3$-Lösung auf pH 8 gestellt und 1 Stunde bei Raumtemperatur gerührt. Die Suspension wird auf 0° abgekühlt und abgenutscht. Das Produkt wird auf Kieselgel chromatographiert (Laufmittel: $CHCl_3$/EtOH/$NH_4OH$ 80:20:1) und aus Aethanol umkristallisiert. Man erhält 2-Amino-5-cyclopropyl-8-fluor-7-(1-piperazinyl)pyrimido[1,6-a]benzimidazol-1,3(2H,5H)-dion. Ausbeute: 43 mg (46%); Smp. 232-234°.

| Mikroanalyse $C_{17}H_{19}FN_6O_2$; 0,3 EtOH: | | | |
|---|---|---|---|
| Ber.: | C 56,70 | H 5,62 | N 22,58 |
| Gef.: | 56,40 | 5,77 | 22,67 |

## Beispiel 13

a) In Analogie zu Beispiel 12a) erhält man aus Aethyl-6-[4-(tert-butoxycarbonyl)-1-piperazinyl]-1-cyclo propyl-5-fluor-2-benzimidazolacetat und tert-Butyl-2-methylcarbazat in einer Ausbeute von 33% das tert-Butyl-4-[2-[[[2-(tert-butoxycarbonyl)-2-methylhydrazino]carbonyl]methyl]-1-cyclopropyl-5-fluor-6-benzimidazolyl]-1-piperazin-carboxylat mit einem Smp. von 180-182°.
MS: 546 (M); 446 [M-(isobuten + $CO_2$)]
b) In Analogie zu Beispiel 12b) erhält man ausgehend von tert-Butyl 4-[2-[[[2-(tert-butoxycarbonyl)-2-methylhydrazino]carbonyl]methyl]-1-cyclopropyl-5-fluor-6-benzimidazolyl]-1-piperazin-carboxylat in einer Ausbeute von 59% das tert-Butyl 7-[4-(tert-butoxycarbonyl)-1-piperazinyl]-5-cyclopropyl-8-fluor-5-dihydro-N-methyl-1,3-dioxopyrimido[1,6-a]benzimidazol-2-carbamat mit einem Smp. von 224-225°.
MS: 573 $(M+H)^+$
c) In Analogie zu Beispiel 12c) erhält man ausgehend von tert-Butyl 7-[4-(tert-butoxycarbonyl)-1-piperazinyl]-5-cyclopropyl-8-fluor-3,5-dihydro-N-methyl-1,3-dioxopyrimido[1,6-a]benzimidazol-2-carbamat in einer Ausbeute von 43% das 5-Cyclopropyl-8-fluor-2-(methylamino)-7-(1-piperazinyl)pyrimido[1,6-a]-benzimidazol-1,3(2H,5H)-dion mit einem Smp. von 228-230°.

| | | | | |
|---|---|---|---|---|
| Mikroanalyse $C_{18}H_{21}FN_6O_2$: | Ber.: | C 58,05 | H 5,68 | N 22,57 |
| | Gef.: | 57,95 | 5,81 | 22,18 |

## Beispiel 14

a) In analogie zu Beispiel 12a) erhält man aus Aethyl-6-[4-(tert-butoxycarbonyl)-1-piperazinyl]-1-cyclopropyl-5-fluor-2-benzimidazolacetat und Hydrazinhydrat das tert-Butyl-4-[2-[[(hydrazino)carbonyl]-methyl]-1-cyclopropyl-5-fluor-6-benzimidazolyl]-1-piperazin-carboxylat. Smp. 172-173°.

| Mikroanalyse $C_{21}H_{29}FN_6O_3$: | Ber.: | C 58,32 | H 6,76 | N 19,43 |
|---|---|---|---|---|
| | Gef.: | 57,86 | 6,91 | 19,20 |

b) Eine Lösung von tert-Butyl-4-[2-[[(hydrazino)carbonyl]methyl]-1-cyclopropyl-5-fluor-6-benzimidazolyl]-1-piperazincarboxylat (0,43 g) in Methanol (22 ml) wird 20 Minuten bei 50° mit einer 35%igen wässrigen Formaldehyd-Lösung (0,102 g) gerührt und anschliessend mit Natriumborhydrid (46 mg) versetzt. Dieses Prozedere wird insgesamt 3 mal durchgeführt. Anschliessend wird das Reaktionsgemisch eingeengt und das Rohprodukt aus Essigester umkristallisiert. Man erhält 0,325 g (70%) tert-Butyl-4-[2-[[[2-(dimethylhydrazino)]carbonyl]-methyl]-1-cyclopropyl-5-fluor-6-benzimidazolyl]-1-piperazin-carboxylat. Smp. 179-181°.
MS: 460 (M)
c) In Analogie zu Beispiel 12b) erhält man ausgehend von tert-Butyl-4-[2-[[[2-(dimethylhydrazino)]-carbonyl]-methyl]1-cyclopropyl-5-fluor-6-benzimidazolyl]-1-piperazin-carboxylat das tert-Butyl 4-[5-cyclopropyl-8-fluor-1,2,3,5-tetrahydro-2-(dimethylamino)-1,3-dioxopyrimido[1,6-a]benzimidazol-7-yl]-1-piperazincarboxylat. Smp. 201-203°.

| Mikroanalyse $C_{24}H_{31}FN_6O_4$: | Ber.: | C 59,25 | H 6,42 | N 17,27 |
|---|---|---|---|---|
| | Gef.: | 59,13 | 7,10 | 17,73 |

d) In Analogie zu Beispiel 12c) erhält man ausgehend von tert-Butyl 4-[5-cyclopropyl-8-fluor-1,2,3,5-tetrahydro-2-(dimethylamino)-1,3-dioxopyrimido[1,6a]benzimidazol-7-yl]-1-piperazincarboxylat das 5-Cyclopropyl-2-(dimethylamino)-8-fluor-7-(1-piperazinyl)pyrimido[1,6-a]benzimidazol-1,3(2H,5H)-dion. Smp. 207-209° (Essigester/Aether).

| Mikroanalyse $C_{19}H_{23}FN_6O_2$: | Ber.: | C 59,06 | H 6,00 | N 21,75 |
|---|---|---|---|---|
| | Gef.: | 58,12 | 6,17 | 20,75 |

## Beispiel A

Es werden in üblicher Weise Gelatinekapseln mit den folgenden Bestandteilen hergestellt:

| | |
|---|---|
| 5-Cyclopropyl-8-fluor-7-(1-piperazinyl)-pyrimido[1,6-a]-benzimidazol-1,3(2H,5H)-dion | 200 mg |
| Luviskol (wasserlösliches Polyvinylpyrrolidon) | 20 mg |
| Mannit | 20 mg |
| Talk | 15 mg |
| Magnesiumstearat | 2 mg |
| | 257 mg |

## Beispiel B

Es werden in üblicher Weise Tabletten mit den folgenden Bestandteilen hergestellt:

| | |
|---|---|
| 5-Cyclopropyl-8-fluor-7-(1-piperazinyl)-pyrimido[1,6-a]-benzimidazol-1,3(2H,5H)-dion | 200 mg |
| Stärke | 44 mg |
| Calciumcarboxymethylcellulose | 30 mg |
| kristalline Cellulose | 40 mg |
| Magnesiumstearat | 6 mg |
| | 320 mg |

## Ansprüche

1. Verbindungen der allgemeinen Formel

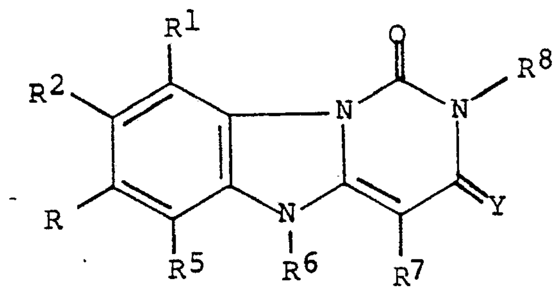

I

worin $R^1$ Wasserstoff, Halogen oder Amino, $R^2$ Halogen, R eine durch niederes Alkyl substituierte 4-Pyridylgruppe oder eine Gruppe $R^3R^4N$-, worin $R^3$ und $R^4$ je Wasserstoff oder niederes Alkyl oder zusammen eine unsubstituierte oder durch niederes Alkyl, Amino, niederes Aminoalkyl, mono- oder di-(niederes Alkyl)amino-niederes Alkyl, Oxo oder die Gruppe -$COOR^a$ oder -$CONR'R''$ substituierte Gruppe der Formel $-(CH_2)_n-X-(CH_2)_m-$ oder $-(CH_2)_p-$, n und m je die Zahl 1, 2 oder 3, wobei n + m höchstens 4 beträgt, p die Zahl 4 oder 5, X ein Sauerstoff- oder Schwefelatom oder die Gruppe -$NR'''$-, $R^a$ Wasserstoff, niederes Alkyl, niederes Alkenyl, Phenyl oder durch Halogen, niederes Alkyl oder Hydroxy mono-, di- oder trisubstituiertes Phenyl, $R'$ und $R''$ je Wasserstoff oder niederes Alkyl, $R'''$ Wasserstoff, Hydroxy, niederes Alkyl oder niederes Aminoalkanoyl, $R^5$ Wasserstoff, Halogen, niederes Alkoxy oder Amino, $R^6$ niederes Alkyl, niederes Cycloalkyl, niederes Halogenalkyl, Phenyl oder durch Halogen, niederes Alkyl, Hydroxy oder niederes Alkoxy mono-, di- oder trisubstituiertes Phenyl, $R^7$ Wasserstoff, niederes Alkyl oder Carboxy, $R^8$ Wasserstoff, Hydroxy, niederes Alkoxy, Amino, niederes Alkylamino oder di-niederes Alkylamino und Y ein Sauerstoff- oder Schwefelatom bedeuten,
und pharmazeutisch annehmbare Salze davon.

2. Verbindungen nach Anspruch 1, worin $R^8$ Wasserstoff, Hydroxy, niederes Alkoxy, Amino oder niederes Alkylamino darstellt.

3. Verbindungen nach Anspruch 2, worin R die Gruppe $R^3R^4N$- und $R^8$ Wasserstoff, Hydroxy oder niederes Alkoxy darstellt.

4. Verbindungen nach Anspruch 3, worin $R^1$ und $R^5$ je Wasserstoff bedeuten.

5. Verbindungen nach Anspruch 3 oder 4, worin $R^2$ Fluor bedeutet.

6. Verbindungen nach einem der Ansprüche 3-5, worin $R^3$ und $R^4$ zusammen eine Gruppe der Formel $-(CH_2)_n-X-(CH_2)_m$ oder eine durch die Gruppe -$COOR^a$ substituierte Gruppe der Formel $(CH_2)_p$-, n und m je die Zahl 2, p die Zahl 4, X die Gruppe -$NR'''$-, $R^a$ niederes Alkyl und $R'''$ Wasserstoff, niederes Alkyl oder niederes Amino- alkanoyl bedeuten.

7. Verbindungen nach Anspruch 6, worin $R^3R^4N$- die 1-Piperazinylgruppe oder die 4-Methyl-1-piperazinylgruppe bedeutet.

8. Verbindungen nach einem der Ansprüche 3-7, worin $R^6$ niederes Alkyl oder niederes Cycloalkyl bedeutet.

9. Verbindungen nach Anspruch 8, worin $R^6$ Aethyl bedeutet.

10. Verbindungen nach Anspruch 8, worin $R^6$ Cyclopropyl darstellt.

11. Verbindungen nach einem der Ansprüche 3-10, worin $R^7$ Wasserstoff oder Carboxy bedeutet.

12. Verbindungen nach einem der Ansprüche 3-11, worin $R^8$ Wasserstoff oder Hydroxy bedeutet.

13. Verbindungen nach einem der Ansprüche 3-12, worin Y ein Sauerstoffatom bedeutet.
14. Verbindungen nach einem der Ansprüche 2-5 und 8-13, worin R die 3,5-Dimethyl-4-pyridylgruppe bedeutet.
15. Verbindungen nach einem der Ansprüche 2-14, worin $R^8$ Amino oder Methylamino bedeutet.
16. Verbindungen nach einem der Ansprüche 1-14, worin $R^8$ Dimethylamino bedeutet.
17. 5-Aethyl-8-fluor-7-(4-methyl-1-piperazinyl)-pyrimido[1,6-a]benzimidazol-1,3(2H,5H)-dion.
18. 5-Cyclopropyl-8-fluor-7-(1-piperazinyl)-pyrimido[1,6-a]benzimidazol-1,3(2H,5H)-dion.
19. 5-Aethyl-9-fluor-3,5-dihydro-8-(4-methyl-1-piperazinyl)-3-thioxopyrimido[1,6a]benzimidazol-1(2H)-on.
20. 5-Cyclopropyl-8-fluor-2-hydroxy-7-(1-piperazinyl)pyrimido[1,6-a]benzimidazol-1,3(2H,5H)-dion.
21. tert-Butyl rac-1-[5-cyclopropyl-8-fluor-2,3-dihydro-1,3-dioxopyrimido[1,6a]benzimidazol-5(1H)-yl]-2-pyrrolidincarboxylat.
22. 7-(4-L-Alanyl-1-piperazinyl)-5-cyclopropyl-8-fluor-2-hydroxypyrimido[1,6-a]benzimidazol-1,3(2H,5H)-dion.
23. 1-[5-Cyclopropyl-8-fluor-1,2,3,5-tetrahydro-2-hydroxy-1,3-dioxopyrimido[1,6-a]benzimidazol-7-yl]-L-prolin-tert-butylester.
24. 5-Aethyl-8-fluor-7-(1-piperazinyl)pyrimido[1,6-a]benzimidazol-1,3(2H,5H)-dion.
25. 5-Aethyl-8-fluor-2-hydroxy-7-(4-methyl-1-piperazinyl)-pyrimido[1,6-a]benzimidazol-1,3(2H,5H)-dion.
26. 5-Cyclopropyl-8-fluor-1,2,3,4-tetrahydro-1,3-dioxo-7-(1-piperazinyl)pyrimido[1,6-a]benzimidazol-4-carbonsäure.
27. 5-Aethyl-8-fluor-2-hydroxy-7-(1-piperazinyl)pyrimido[1,6-a]benzimidazol-1,3(2H,5H)-dion.
28. 2-Amino-5-cyclopropyl-8-fluor-7-(1-piperazinyl)pyrimido[1,6-a]benzimidazol-1,3(2H,5H)-dion.
29. 5-Cyclopropyl-8-fluor-2-(methylamino)-7-(1-piperazinyl)pyrimido[1,6-a]benzimidazol-1,3(2H,5H)-dion.
30. 5-Cyclopropyl-2-(dimethylamino)-8-fluor-7-(1-piperazinyl)pyrimido[1,6-a]benzimidazol-1,3(2H,5H)-dion.
31. Verbindungen der allgemeinen Formel

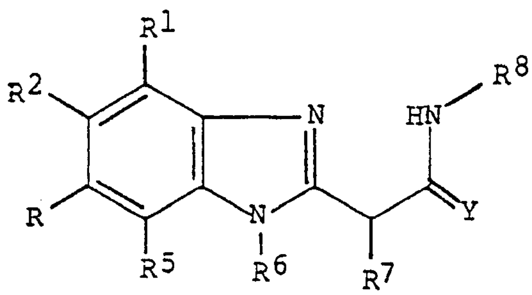

II

worin R, Y, $R^1$, $R^2$, $R^5$, $R^6$, $R^7$ und $R^8$ die in Anspruch 1 angegebene Bedeutung besitzen, wobei allfällig vorhandene freie Hydroxy-, Amino- und Carboxygruppen in geschützter Form vorliegen.
32. Verbindungen der allgemeinen Formel

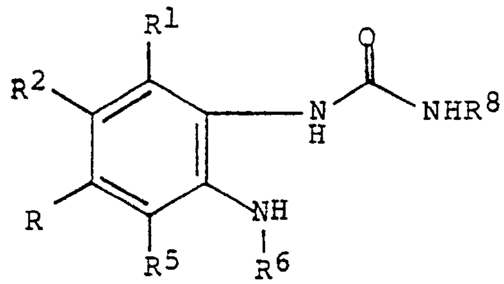

IV

worin R, $R^1$, $R^2$, $R^5$, $R^6$ und $R^8$ die in Anspruch 1 angegebene Bedeutung besitzen, wobei allfällig vorhandene freie Hydroxy-, Amino- und Carboxy- gruppen in geschützter Form vorliegen.
33. Verbindungen der allgemeinen Formel

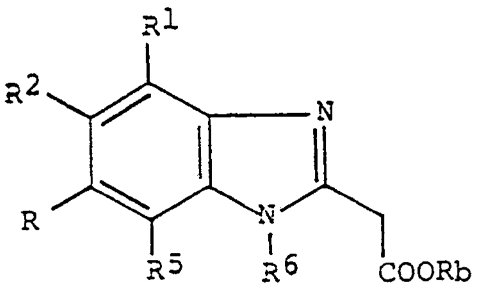

VI

worin Rb eine Carboxy-Schutzgruppe bedeutet, und R, $R^1$, $R^2$, $R^5$ und $R^6$ die in Anspruch 1 angegebene Bedeutung besitzen, wobei allfällig vorhandene freie Hydroxy-, Amino- und Carboxygruppen in geschützter Form vorliegen.

34 Verbindungen der Formel I in Anspruch 1, welche mindestens eine geschützte Hydroxy-, Amino- und/oder Carboxygruppe besitzen.

35. Verbindungen nach einem der Ansprüche 1-30 zur Anwendung als therapeutische Wirkstoffe.

36. Verbindungen nach einem der Ansprüche 1-30 zur Anwendung als antibakteriell wirksame Stoffe.

37. Verfahren zur Herstellung von Verbindungen nach einem der Ansprüche 1-30, dadurch gekennzeichnet, dass man

a) eine Verbindung der allgemeinen Formel

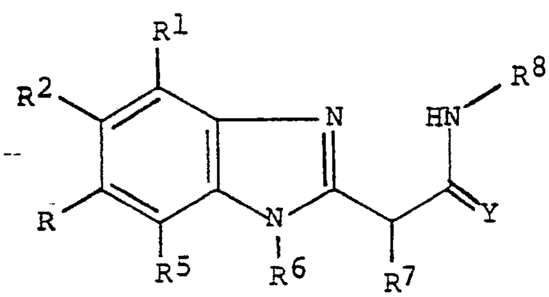

II

worin R, Y, $R^1$, $R^2$, $R^5$, $R^6$, $R^7$ und $R^8$ die in Anspruch 1 angegebene Bedeutung besitzen, wobei allfällig vorhandene freie Hydroxy-, Amino- und Carboxygruppen in geschützter Form vorliegen,
gegebenenfalls in Gegenwart einer Base mit einer Verbindung der allgemeinen Formel

X-CO-X III

worin X eine Abgangsgruppe bedeutet,
umsetzt, oder

b) eine Verbindung der allgemeinen Formel

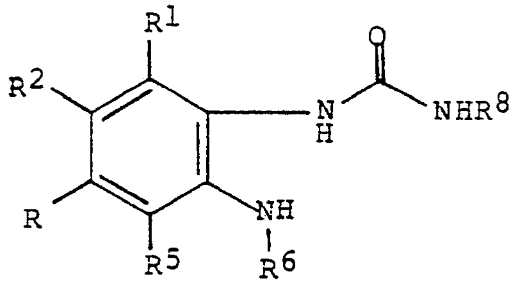

IV

worin R, $R^1$, $R^2$, $R^5$, $R^6$ und $R^8$ die in Anspruch 1 angegebene Bedeutung besitzen, wobei allfällig vorhandene freie Hydroxy-, Amino- und Carboxygruppen in geschützter Form vorliegen,
in Gegenwart einer Base mit einer Verbindung der allgemeinen Formel

$RaOOC-CHR^7-COORa$ V

worin Ra niederes Alkyl bedeutet, und $R^7$ die in Anspruch 1 angegebene Bedeutung besitzt, wobei eine allfällig vorhandene freie Carboxygruppe in geschützter Form vorliegt,

umsetzt, oder

c) eine Verbindung der allgemeinen Formel

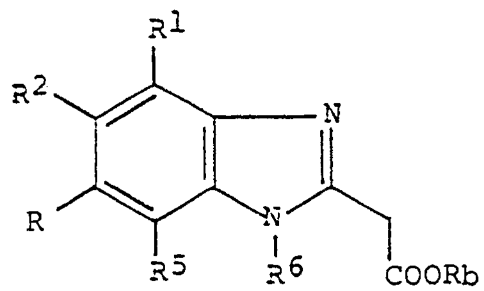

VI

worin Rb eine Carboxy-Schutzgruppe bedeutet, und R, $R^1$, $R^2$, $R^5$ und $R^6$ die in Anspruch 1 angegebene Bedeutung besitzen, wobei allfällig vorhandene freie Hydroxy-, Amino- und Carboxygruppen in geschützter Form vorliegen,

mit 2 Mol-Aequivalenten Chloracetylisocyanat umsetzt, worauf man allfällig vorhandene Schutzgruppen abspaltet und eine erhaltene Verbindung der Formel I erwünschtenfalls in ein pharmazeutisch annehmbares Salz überführt.

38. Arzneimittel, enthaltend eine Verbindung gemäss einem der Ansprüche 1-30 und einen therapeutisch inerten Träger.

39 Antibakteriell wirksame Mittel, enthaltend eine Verbindung gemäss einem der Ansprüche 1-30 und einen therapeutisch inerten Träger.

40. Verwendung von Verbindungen gemäss einem der Ansprüche 1-30 bei der Bekämpfung oder Verhütung von Krankheiten.

41. Verwendung von Verbindungen gemäss einem der Ansprüche 1-30 bei der Bekämpfung oder Verhütung von bakteriellen Infektionen.

42. Verwendung von Verbindungen gemäss einem der Ansprüche 1-30 bei der Herstellung von antibakteriell wirksamen Mitteln.

43. Verbindungen nach Anspruch 1, worin $R^1$, $R^5$ und $R^7$ Wasserstoff, R 4-Methyl-1-piperazinyl, $R^6$ Cyclopropyl, $R^8$ Hydroxy und Y Sauerstoff darstellt.

Europäisches Patentamt

**EUROPÄISCHER TEILRECHERCHENBERICHT,**
der nach Regel 45 des Europäischen Patent-übereinkommens für das weitere Verfahren als europäischer Recherchenbericht gilt

Nummer der Anmeldung

EP 90121665.5

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | JOURNAL OF HETEROCYCLIC CHEMISTRY, Band 26, 1989 E.A.M. BADAWEY et al. "Benzimidazole Condensed Ring Systems 4(1). New Approaches to the Synthesis of Substituted Pyrimido (1,6-a)benzimidazole-1,3(2H, 5H)-diones" Seiten 405-408 * Seite 406 * -- | 1-31, 33-39, 42 | C 07 D 487/04 C 07 C 275/40 C 07 D 235/16// (C 07 D 487/04 C 07 D 239:00 C 07 D 235:00) |
| A | JOURNAL OF HETEROCYCLIC CHEMISTRY, Band 26, 1989 E.A.M. BADAWEY et al. "Benzimidazole Condensed Ring Systems 5(1), Studies on the Synthesis of Pyrimido (1,6-a)benzimidazole-1,3(2H, 5H)-diones" Seiten 1401-1404 * Seiten 1401,1402 * -- | 1-31, 33-39, 42 | RECHERCHIERTE SACHGEBIETE (Int. Cl.5) |
| A | JOURNAL OF THE CHEMICAL | 1-30, | C 07 D 487/00 C 07 D 235/00 C 07 C 275/00 |

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-39, 42
Unvollständig recherchierte Patentansprüche: -
Nicht recherchierte Patentansprüche: 40, 41 (Verfahren zur
Grund für die Beschränkung der Recherche:
therapeutischen Behandlung des menschlichen oder tierischen Körpers (Art. 52(4) EPÜ))

| Recherchenort | Abschlußdatum der Recherche | Prufer |
|---|---|---|
| WIEN | 25-02-1991 | PETROUSEK |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

Europäisches Patentamt

**EUROPÄISCHER TEILRECHERCHENBERICHT**

Nummer der Anmeldung
-2-
EP 90121665.5

| EINSCHLÄGIGE DOKUMENTE | | | KLASSIFIKATION DER ANMELDUNG (Int. Cl.⁵) |
|---|---|---|---|
| Kategorie | Kennzeichnung des Dokuments mit Angabe, soweit erforderlich, der maßgeblichen Teile | betrifft Anspruch | |
| | SOCIETY, 1965<br>H.J. DAVIES et al.<br>"Pyrimido(3,4-a)benzimidazole : A Novel Ring System"<br>Seiten 5125-5127<br>* Seiten 5125,5126 *<br>-- | 32,<br>34-39,<br>42 | |
| A | DE - A1 - 2 930 333<br>(BASF AG)<br>* Ansprüche 1,2; Seite 8 *<br>-- | 1-31,<br>33,34,<br>37 | |
| A | EP - A1 - 0 012 983<br>(HOECHST AKTIENGESELLSCHAFT)<br>* Anspruch 1 *<br>-- | 32 | |
| A | US - A - 4 209 523<br>(L. LAFON)<br>* Anspruch 1 *<br>---- | 31 | RECHERCHIERTE SACHGEBIETE (Int. Cl.⁵) |